(19) Europäisches Patentamt
European Patent Office
Office européen des brevets

(11) Publication number: **0 000 609**
**B1**

(12) **EUROPEAN PATENT SPECIFICATION**

(45) Date of publication of patent specification: **23.09.81**

(21) Application number: **78200112.7**

(22) Date of filing: **20.07.78**

(51) Int. Cl.[3]: **C 07 J 5/00,** C 07 J 71/00, C 07 J 21/00, A 61 K 31/57, A 61 K 31/58

(54) **Novel 19-nor-pregnahexaenes, process for the preparation thereof, and pharmaceutical compositions containing them.**

(30) Priority: **26.07.77 US 819182**

(43) Date of publication of application:
**07.02.79 Bulletin 79/3**

(45) Publication of the grant of the European patent:
**23.09.81 Bulletin 81/38**

(84) Designated Contracting States:
**BE CH DE FR GB NL SE**

(56) References cited:
**US - A - 3 410 879**

**JOURNAL OF THE AMERICAN CHEMICAL SOCIETY, vol. 89 (1967) pages 1919—1925**

(73) Proprietor: **SCHERICO LTD.**
**Töpferstrasse 5**
**CH-6004 Lucerne (CH)**

(72) Inventor: **Casmer, Charles Jesse**
**383 Berthold Avenue**
**Rahway New Jersey 07065 (US)**
Inventor: **Draper, Richard William**
**17 Skyline Drive**
**North Caldwell New Jersey 07006 (US)**

(74) Representative: **Antony, Fritz, Dr. et al,**
**P.O. Box 601 Winkelriedstrasse 35**
**CH-6002 Lucerne (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 000 609 B1

Courier Press, Leamington Spa, England.

Novel 19-nor-pregnahexaenes, process for the preparation thereof, and pharmaceutical compositions containing them

This invention relates to 19-nor-pregna-1,3,5(10),6,8,14-hexaene-20-ones, to a process for their preparation, and to pharmaceutical compositions containing them.

The invention is based upon the observation that certain 19-nor-pregna-1,3,5(10),6,8,14-hexaene-20-ones, which can be defined by the general formula (I) as set forth hereinbelow, possess anti-mitotic activity with minimal or no hormonal side effects and are, thus, useful in the treatment of diseases characterized by rapid and/or abnormal cell proliferation, particularly in the treatment and control of psoriasis.

In general, the compounds of formula (I) are novel compounds, although at least one compound embraced by such formula, viz. 19-nor-pregna-1,3,5(10),6,8,14-hexaene-3,17$\alpha$,21-triol-20-one 3,21-diacetate [and possibly, by implication, its 3-(free hydroxy) analog], is described in the prior art [Heller et al., J. Am. Chem. Soc. 89, 1919 et sequ. (1967)]. The therapeutic activity thereof, or of related compounds, is, however, neither disclosed nor suggested by the prior art.

Accordingly, the active compounds of the novel therapeutic compositions of the invention are defined by the following general formula

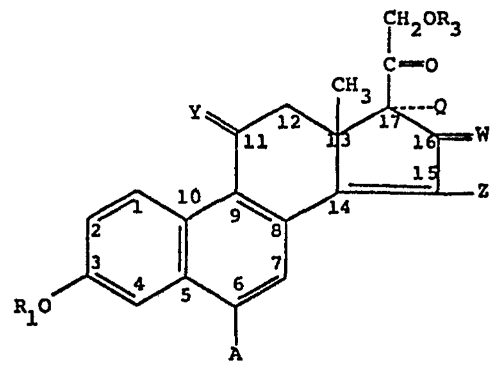

(I),

wherein

A is hydrogen, lower alkyl, fluoro or fluoro-substituted methyl;

$R_1$ is hydrogen, lower alkyl, or an acyl radical of a carboxylic acid having up to 12 carbon atoms;

W is (H,H); (H, lower alkyl); (H, $\alpha$-$OR_2$), with $R_2$ being hydrogen or an acyl radical of a carboxylic acid having up to 12 carbon atoms; or =CHT, with T being hydrogen, lower alkyl, fluorine, or chlorine;

Q is $OR_4$ (with $R_4$ being hydrogen or an acyl radical of a carboxylic acid having up to 12 carbon atoms); hydrogen, provided W is (H,H), or (H, lower alkyl); or together with W represents a 16$\alpha$,17$\alpha$-lower alkylidenedioxy grouping;

Y is (H,H), (H,OH), or oxygen;

Z is hydrogen, chlorine or bromine;

$R_3$ is hydrogen or an acyl radical of a carboxylic acid having up to 12 carbon atoms; or $OR_3$ together with Q represents an alkylidenedioxy or alkylorthoalkanoate grouping; and when Q is hydroxy and $R_3$ is hydrogen, the 17$\alpha$, 20; 20, 21-bismethylenedioxy derivatives thereof.

Claimed as per-se compounds of the invention are the compounds of general formula (I) with the exception of the 21-acetate and 3,21-diacetate of 19-nor-pregna-1,3,5(10),6,8,14-hexaene-3,17$\alpha$,21-triol-20-one.

Lower alkyl groups included within the definitions of A, $R_1$ and W are preferably those having up to four carbon atoms such as methyl, ethyl, n-propyl, isopropyl, n-butyl, sec.-butyl, and tert.-butyl, although higher homologs such as pentyl and hexyl fall within the scope of this invention.

The acyl radicals of carboxylic acids having up to twelve carbon atoms included within the definitions of $R_1$, $R_2$, $R_3$ and $R_4$ may be saturated or unsaturated, straight-chain or branched-chain aliphatic, cycloaliphatic or cycloaliphatic-aliphatic, aromatic, aryl-aliphatic, or alkyl-aromatic, and may be substituted, e.g., by hydroxy, aryloxy, alkoxy containing from 1 to 5 carbon atoms or halogen. Typical ester groups of the 19-nor-pregnahexaenes of the formulations of our invention are thus derived from carboxylic acids such as alkanoic acids exemplified by acetic, propionic, trimethylacetic, butyric, isobutyric, valeric, isovaleric, caproic, tert.-butylacetic, enanthic, caprylic, capric, cyclopentylpropionic, undecylic, lauric, and adamantanecarboxylic acids; substituted alkanoic acids such as phenoxyacetic, trifluoroacetic, and $\beta$-chloropropionic and $\beta$-benzoylaminoisobutyric acids; from aromatic acids including benzoic, toluic, $p$-chlorobenzoic, $p$-fluorobenzoic, $p$-methoxybenzoic, and 3',5'-dimethylbenzoic acids; aryl-alkanoic acids such as phenylacetic and phenylpropionic acids; unsaturated acids such as acrylic and sorbic acids; and dibasic acids such as succinic, tartaric and phthalic acids.

Preferred acyl radicals as defined by $R_1$, $R_2$, $R_3$ and $R_4$ in formula (I) are those derived from lower alkanoic acids, having preferably up to 8 carbon atoms, such as radicals obtained from acetic,

propionic, butyric, valeric, caprylic, caproic, tert.-butylacetic acid and the like, as well as acyl radicals derived from aromatic carboxylic acids having up to 8 carbon atoms, preferably from benzoic acid.

The alkylidene groups contemplated in the compounds of our invention are preferably lower alkylidenes, i.e. hydrocarbon radicals having preferably up to 4 carbon atoms including radicals such as methylene, ethylidene, n-propylidene, isopropylidene, n-butylidene, and sec.-butylidene.

The 19-nor-pregnahexaene-20-ones of this invention are crystalline solids, usually white to off-white in color, which are insoluble in water and soluble in most organic solvents, particularly in acetone, dioxane, dimethylformamide, and dimethylsulfoxide, although of limited solubility in non-polar solvents such as dialkylethers and alkylhydrocarbons.

The 19-nor-pregnahexaene-20-ones of formula (I) exhibit anti-mitotic activity and, in particular, are useful in the treatment and control of psoriasis.

Useful 19-nor-pregnahexaene-20-ones of formula (I) include 16α-methyl-19-nor-pregna-1,3,5(10),6,8,14-hexaene-3,17α,21-triol-20-one 21-acetate and the 6-methyl, 6-fluoro, 6-difluoromethyl and 6-trifluoro derivatives thereof, as well as the 16-desmethyl analogs and the 16β-methyl epimers thereof; 16α-hydroxy-substituted compounds of formula (I) and ester and 16α,17α-alkylidenedioxy derivatives thereof such as 19-nor-pregna-1,3,5(10),6,8,14-hexaene-3,16α,17α,21-tetrol-20-one 16,21-diacetate and 16α,17α-isopropylidenedioxy-19-nor-pregna-1,3,5,(10),6,8,14-hexaene-3,21-diol-20-one 21-acetate; 16-alkylidene-substituted compounds of formula I such as 16-methylene-19-nor-pregna-1,3,5(10),6,8,14-hexaene-3,17α,21-triol-20-one 21-acetate and the 3-acetate and 3-methyl ether derivatives thereof; and the 15-chloro derivatives of the foregoing.

Of the compounds of formula I, especially useful for the treatment of psoriasis are the 16α-alkylsubstituted compounds [i.e. compounds of formula (I) wherein W is (H, α-alkyl)], preferred compounds being the 16α-methyl-19-nor-pregna-1,3,5(10),6,8,14-hexaene-20-ones of the general formula

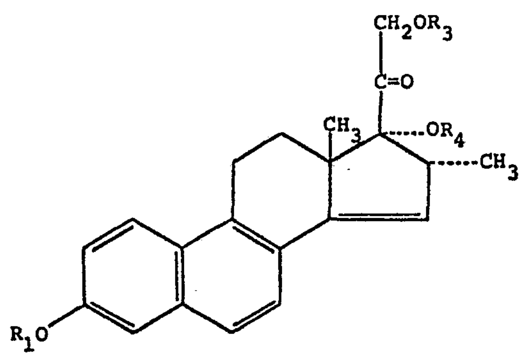

(II),

wherein

$R_1$, $R_3$ and $R_4$ are as defined above for formula (I).

Among the compounds of formula (II), particularly useful anti-psoriatic agents are those wherein $R_4$ is hydrogen and, of these, especially those wherein $R_3$ is hydrogen or acetyl. Of the foregoing, a particularly preferred species is 16α-methyl-19-nor-pregna-1,3,5(10),6,8,14-hexaene-3,17α,21-triol-20-one 21-acetate, which exhibits superior anti-mitotic activity at topical doses as low as 20 micrograms when administered topically to mice in which epidermal mitosis has been stimulated by prior application of croton oil. Further preferred compounds of formula II include the 3-acetate and 3-benzoate ester and the 3-methyl ether derivatives of the former; 16α-methyl-19-nor-pregna-1,3,5(10),6,8,14-hexaene-3,17α,21-triol-20-one; and the 21-propionate, 17-propionate and 17,21-di-n-butyrate ester derivatives of the latter.

The 19-nor-pregnahexaene-20-ones of formula I are conveniently prepared from the corresponding 19-nor-pregna-1,3,5(10),6,8-pentaene-20-ones by dehydrogenation in position 14, most suitably by reaction with a molar equivalent of 2,3-dichloro-5,6-dicyanobenzoquinone (DDQ) in an aprotic solvent (usually dioxane) in an essentially neutral medium. — Alternatively, an appropriate 11-unsubstituted pregna-1,4,6,8,14-pentaene-3-one can be subjected to aromatization — e.g. by means of a weak base, desirably in the presence of a soluble halide salt such as lithium chloride —, or an appropriate 9α,11β-dihalogeno-pregna-1,4,6-triene-3-one can be subjected to concomitant didehydrohalogenation and aromatization (a specific embodiment being concomitant 6-dehydrogenation, didehydrochlorination and aromatization of an appropriate 9α,11β-dichloro-pregna-1,4-diene-3-one, suitably in situ, at elevated temperatures, by means of DDQ as dehydrogenating agent and in the presence of an acid in an aprotic solvent). — Isolation of the respective 19-nor-pregnahexaene-20-ones is then effected by methods well known in the steroid art.

When the above 14-dehydrogenation of a 19-nor-pregna-1,3,5(10),6,8-pentaene-20-one precursor is carried out in the presence of at least a molar equivalent of hydrogen chloride and with about two molar equivalents of DDQ, there are formed the respective 15-chloro-19-nor-pregnahexaene-20-ones of formula (I). Thus, for example, reaction of 16α-methyl-19-nor-pregna-

1,3,5(10),6,8-pentaene-3,17α,21-triol-20-one 21-acetate in dioxane with at least a molar equivalent of hydrogen chloride and with about two molar equivalents of DDQ yields 15-chloro-16α-methyl-19-nor-pregna-1,3,5(10),6,8,14-hexaene-3,17α,21-triol-20-one 21-acetate, having anti-mitotic activity. — Similarly, by substituting hydrogen bromide for hydrogen chloride, the respective 15-bromo compounds are obtained. — Alternatively, a 15-chloro or -bromo substituent may be introduced by reacting the respective 15-unsubstituted 1,3,5(10),6,8,14-hexaene with halogenating agents such as molecular chlorine or bromine, or N-halo-imides (e.g. N-halosuccinimide), or hydrogen halide in the presence of DDQ.

The foregoing process utilizing hydrogen chloride and DDQ for preparing the 15-chloro-substituted compounds of this invention is preferably carried out at room temperature (although temperatures in the range of from about 0° to 100°C may be employed) and in dioxane (although other aprotic solvents may be used, particularly ethers such as tetrahydrofuran, diethylether and diglyme). When carried out at room temperature, the reaction is usually complete in 30 minutes as determined by thin layer chromatography, although at lower temperatures it may take up to 24 hours before complete conversion of a 19-nor-pregna-1,3,5(10),6,8-pentaene-20-one to the corresponding 15-chloro-14-dehydro compound has been effected. Although, in our process, only a molar equivalent of hydrogen chloride is required per mole of the pregnapentaene-20-one starting compound, we prefer to use large excesses of hydrogen chloride (e.g. a saturated solution of hydrogen chloride in dioxane) since the rate of reaction is thereby increased and the process is completed in thirty minutes or less.

Many of the 19-nor-pregna-1,3,5(10),6,8-pentaene-20-one intermediates from which the 19-nor-pregnahexaene-20-ones of this invention may be obtained are known in the art (e.g. described in U.S. Patent Specifications Nos. 3,182,057 and 3,182,075) and have been prepared by reaction of a 9α-11β-dichloro-1,4-pregnadiene-17α,21-diol-3,20-dione 21-alkanoate (e.g. 16α-methyl-9α-11β-dichloro-1,4-pregnadiene-17α,21-diol-3,20-dione 21-acetate) with a weak base, preferably in the presence of lithium chloride. Weak bases useful in this process are pyridine, collidine, lutidine and, preferably, dimethylformamide. Other 19-nor-pregnapentaene-20-one intermediates may also be prepared from the corresponding 9α,11β-dichloro-1,4-pregnadiene-3,20-diones in similar manner.

The 9α,11β-dichloro-1,4-pregnadiene-17α,21-diol-3,20-dione precursors to the 19-nor-pregna-1,3,5(10),6,8-pentaene-20-one intermediates are also known in the art and may be prepared from the corresponding 9(11)-dehydro derivatives according to procedures such as described in U.S. Patents Nos. 3,894,963 and 3,009,933.

When preparing a 16-alkylidene compound of formula (I) (i.e. a compound wherein W is =CHT), one may start with a 9α,11β-dichloro-16-alkylidene-1,4-pregnadiene-17α,21-diol-3,20-dione 21-lower alkanoate precursor and convert it to a 16-alkylidene-19-nor-pregna-1,3,5(10),6,8-pentaene and thence to the 14-dehydro analog of formula (I) according to the process described hereinabove. — Alternatively, to minimize side reactions which occur when halogenating a 16-methylene-17α-hydroxy-1,4,9(11)-pregnatriene-3,20-dione, one may protect the 17α-hydroxyl function thereof, e.g. by esterification, after introduction of the 9(11)-double bond. After preparing the corresponding 9α,11β-dichloro derivative of the 17α-hydroxy-protected derivative of a 16-methylene-1,4,9(11)-pregnatriene-3,20-dione (e.g. 16-methylene-9α,11β-dichloro-1,4-pregnadiene-17α,21-diol-3,20-dione 17,21-diacetate) and thence conversion thereof to a 16-alkylidene-19-nor-pregna-1,3,5(10),6,8,14-hexaene-3-ol of formula (I) [e.g. 16-methylene-19-nor-pregna-1,3,5(10),6,8,14-hexaene-3,17α,21-triol-20-one 17,21-diacetate], the 17α-hydroxy protecting groups may be easily removed via known techniques (e.g. by means of aqueous sodium bicarbonate in methanol), to obtain a 16-alkylidene-19-nor-pregna-1,3,5(10),6,8,14-hexaene-3,17α,21-triol-20-one of this invention [e.g. 16-methylene-19-nor-pregna-1,3,5(10),6,8,14-hexaene-3,17α,21-triol-20-one].

When converting a 19-nor-pregna-1,3,5(10),6,8-pentaene-20-one to the corresponding 19-nor-pregna-1,3,5(10),6,8,14-hexaene-20-one by reaction with DDQ as described hereinabove, it is often necessary that the 21-hydroxyl group and any 16-hydroxyl group which may be present be protected, such as by an acyl function. It is preferred to utilize lower alkanoate ester derivatives (usually acetates) of the 19-nor-pregna-pentaene-20-one intermediates, thereby producing the 19-nor-pregnahexaene-20-ones of formulae (I) and (II) as 21-alkanoates, usually 21-acetates; the corresponding 21-free-hydroxy compound is then easily obtained from the 21-alkanoate via known hydrolytic procedures, such as with aqueous sodium bicarbonate in methanol or by utilizing diastase enzyme of malt in aqueous ethanol using known procedures.

In general, when a 21-mono-lower alkanoate or a 17,21-dilower alkanoate derivative of a 3-(free-hydroxy)-19-nor-pregna-hexaene-20-one of formula (I) is desired, it is preferable to use as starting compound a 19-nor-pregna-1,3,5(10),6,8-pentaene-20-one intermediate containing the desired 21-monoalkanoate or 17,21-di-alkanoate ester function prior to reaction with DDQ.

A 17-mono-lower alkanoate ester derivative of a 3-(free hydroxy)-19-nor-pregnahexaene-20-one of formula (I) may be prepared by reaction of the respective 17-free-hydroxy compound [e.g. 16α-methyl-19-nor-pregna-1,3,5(10),6,8,14-hexaene-3,17α,21-triol-20-one] in an aprotic solvent (e.g. dimethylsulfoxide) with at least one molar equivalent of a tri-lower alkyl orthoester (e.g. triethylortho-propionate) in the presence of a strong acid (e.g. *p*-toluenesulfonic acid) followed by hydrolytic cleavage of the resulting 17α,21-orthoester by means of aqueous acid (e.g. aqueous acetic acid),

thence separation and isolation of the 17-mono-ester using known techniques, usually including chromatographic methods, whereby there is obtained a 17-mono-alkanoate (e.g. the 17-propionate). By this procedure, there is usually also produced some of the corresponding 21-mono-alkanoate derivatives (e.g. the 21-propionate) which may also be isolated via chromatographic techniques.

A 3,17-diester derivative of formula (I) is conveniently prepared from a corresponding 3-(free-hydroxy)-17$\alpha$,21-orthoester (obtainable as described hereinabove) by reaction thereof with an acid anhydride or acid halide in pyridine (e.g. acetic anhydride in pyridine) to form the corresponding 3-(esterified-hydroxy)-17$\alpha$,21-orthoester, which, after hydrolytic cleavage of the 17$\alpha$-21-orthoester group by means of aqueous acetic acid, yields a 3,17-diester of formula (I).

The 3,21-diester derivatives of formula (I) are conveniently prepared from the corresponding 21-monoesters; the 3,17$\alpha$,21-triesters may be prepared from the corresponding 17$\alpha$,21- or 3,17$\alpha$-diesters utilizing conventional esterification techniques.

To prepare a 3-monoester derivative of formula (I) it is often necessary to protect the 21-hydroxyl group (e.g. by an ether derivative such as the 21-methoxyethoxymethyl ether) in the 9$\alpha$,11$\beta$-dichloro-1,4-pregnadiene-3,21-dione precursor (e.g. by reaction of 16$\alpha$-methyl-9$\alpha$,11$\beta$-dichloro-1,4-pregnadiene-17$\alpha$,21-diol-3,20-dione with N,N,N-triethyl-N-methoxyethoxymethyl-ammonium chloride in acetonitrile) prior to reaction thereof with a weak base in the presence of lithium chloride to produce the corresponding 3-(free-hydroxy)-19-nor-pregna-1,3,5(10),6,8-pentaene-20-one [e.g. 16$\alpha$-methyl-19-nor-pregna-1,3,5(10),6,8-pentaene-3,17$\alpha$,21-triol-20-one 21-methoxyethoxymethyl ether]. Reaction of such 21-protected 19-nor-pregna-1,3,5(10),6,8-pentaene-20-one with DDQ yields the corresponding 19-nor-pregna-1,3,5(10),6,8,14-hexaene-20-one [e.g. 16$\alpha$-methyl-19-nor-pregna-1,3,5(10),6,8,14-hexaene-3,17$\alpha$,21-triol-20-one 21-methoxyethoxymethyl ether], which, upon treatment thereof according to standard esterification procedures (e.g. by reaction with acetic anhydride in pyridine), yields the corresponding 3-monoester derivative. Upon deprotection of the 21-position (e.g. cleavage of the 21-ether function by means of zinc bromide in methylene-chloride), there is then produced the desired 3-monoester of formula (I) [e.g. 16$\alpha$-methyl-19-nor-pregna-1,3-5(10)-6,8,14-hexaene-3,17$\alpha$,21-triol-20-one 3-acetate].

The 3-alkoxy derivatives of formula (I) are conveniently prepared via known etherification techniques such as those utilizing a diazoalkane (e.g. diazomethane in ether). Thus, a 3-alkoxy-21-monoester or a 3-alkoxy-17,21 diester derivative is prepared from the corresponding 3-hydroxy-21-monoester or 3-hydroxy-17,21-diester derivative, respectively, by reaction with a diazoalkane in ether. — A derivative of formula (I) having a 3-alkoxy group and free hydroxyl functions at 17 and 21 may be conveniently obtained from a 3-alkoxy-21-monoester derivative via hydrolysis such as with aqueous sodium bicarbonate in methanol. — In order to prepare a 3-alkoxy-17-monoester derivative of a compound of formula (I) [e.g. 16$\alpha$-methyl-19-nor-pregna-1,3,5(10),6,8,14-hexaene-3,17$\alpha$,21-triol-20-one 17-acetate 3-methyl ether] it is preferable to first prepare a 17$\alpha$,21-orthoester derivative of a 3,17$\alpha$,21-triol of formula (I) according to procedures described hereinabove, followed by reaction thereof with a diazoalkane (e.g. diazomethane) to produce the corresponding 3-alkoxy-17$\alpha$,21-orthoester derivative, followed by cleavage of the 17$\alpha$,21-orthoester grouping by means of dilute acid to obtain the desired 3-alkoxy-17-monoester derivative of formula (I).

When preparing a 16$\alpha$,17$\alpha$-alkylidenedioxy derivative of formula (I), the 16$\alpha$,17$\alpha$-alkylidene-dioxy function may be introduced into the molecule after preparation of the corresponding 16$\alpha$,17$\alpha$-di-(free-hydroxy)-19-nor-pregna-1,3,5(10),6,8,14-hexaene-20-one or at an earlier stage of the synthesis; however, a 17$\alpha$,21-alkylidenedioxy grouping is preferably introduced after preparation of the corresponding 17$\alpha$,21-di-(free-hydroxy)-19-nor-pregna-1,3,5(10),6,8,14-hexaene-20-one. Both, the 16$\alpha$,17$\alpha$- and the 17$\alpha$,21-alkylidenedioxy derivatives of the 19-nor-pregna-1,3,5(10),6,8,14-hexaene-20-ones of formula (I) may be prepared from the corresponding 16$\alpha$,17$\alpha$-di-(free-hydroxy)- or 17$\alpha$,21-di(free-hydroxy)-steroids upon reaction with a ketone or aldehyde (e.g. acetone, acetaldehyde, acetophenone) in the presence of a mineral acid (e.g. hydrochloric acid). The 17$\alpha$,20; 20,21-bismethylenedioxy function can be introduced prior to or after introduction of the 19-nor-pregna-pentaene or 19-nor-pregnahexaene system by known reactions such as that utilizing formaldehyde in the presence of acid.

The following Examples illustrate the invention:

## EXAMPLE 1

### 16$\alpha$-METHYL-19-NOR-PREGNA-1,3,5(10),6,8,14-HEXAENE-3,17$\alpha$,21-TRIOL-20-ONE 21-ACETATE

A. 16$\alpha$-Methyl-19-nor-pregna-1,3,5(10),6,8-pentaene-3,17$\alpha$,21-triol-20-one 21-Acetate

To a refluxing solution of lithium chloride (120 g) and concentrated hydrochloric acid (1.8 ml) in dimethylformamide (750 ml), add 9$\alpha$,11$\beta$-dichloro-16$\alpha$-methyl-1,4-pregna-diene-17$\alpha$,21-diol-3,20-dione 21-acetate (30 g). Heat the reaction mixture at reflux temperature for 15 minutes, then pour into water/ice (6 liters). Extract the aqueous mixture with ethyl acetate, wash the combined extracts with water, then evaporate to a volume of about 350 ml. Separate the resultant crystalline precipitate by filtration, wash the precipitate with ethyl acetate and air dry, to obtain 16$\alpha$-methyl-19-nor-pregna-1,3,5(10),6,8-pentaene-3,17$\alpha$,21-triol-20-one 21-acetate (yield 9.6 g); m.p. = 235—240°C; $[\alpha]_D^{26}$

= +101° (dioxane $\lambda_{max}^{methanol}$ [in nm] = 230 ($\varepsilon$ = 81,100), 258 ($\varepsilon$ = 3600), 269 ($\varepsilon$ = 4900), 280 ($\varepsilon$ = 5600), 292 ($\varepsilon$ = 4100), 326 $\varepsilon$ = 2400), 346 ($\varepsilon$ = 800).

B. 16α-Methyl-19-nor-pregna-1,3,5(10),6,8,14-hexaene-3,17α,21-triol-20-one 21-Acetate

To a solution of 16α-methyl-19-nor-pregna-1,3,5(10),6,8-pentaene-3,17α,21-triol-20-one 21-acetate (14.0 g) in dioxane (2 liters), add 2,3-dichloro-5,6-dicyanobenzoquinone (9.98 g = 1.2 equivalents) and stir the reaction mixture at room temperature for 4 1/2 hours. Separate the precipitated solids by filtration and wash the precipitate with dioxane. Combine the filtrate and washings and evaporate to a small volume. Dissolve the residue in ethyl acetate, wash the ethyl acetate solution with water, then with aqueous sodium bicarbonate solution, thereafter with saturated sodium chloride solution, and then again with water. Evaporate the ethyl acetate solution *in vacuo* to a small volume and separate the resultant precipitate by filtration, to obtain 16α-methyl-19-nor-pregna-1,3,5(10),6,8,14-hexaene-3,17α,21-triol-20-one 21-acetate (yield 6.48 g). Concentrate the filtrate to dryness, triturate the resultant residue with ether and filter, to obtain an additional 4.47 g of 16α-methyl-19-nor-pregna-1,3,5(10),6,8,14-hexaene-3,17α,21-triol-20-one 21-acetate; $[\alpha]_D^{26}$ = −95° (dioxane), m.p. = 218—221°C; $\lambda_{max}^{methanol}$ [in nm] = 248 ($\varepsilon$ = 36,000), 257 ($\varepsilon$ = 46,400), 266 ($\varepsilon$ = 49,200), 288 (shoulder) ($\varepsilon$ = 13,900), 298 ($\varepsilon$ = 19,000), 310 ($\varepsilon$ = 17,900).

## EXAMPLE 2.
## OTHER 19-NOR-PREGNA-1,3,5(10),6,8,14-HEXAENE-3,17α,21-TRIOL-20-ONE DERIVATIVES

A. 19-Nor-pregna-1,3,5(10),6,8-pentaene-3,17α,21-triol-20-one Derivatives

In a manner similar to that described in Example 1A, treat each of the following 9α,11β-dihalogeno-1,4-pregna-dienes with lithium chloride in dimethylformamide:

1) 9α,11β-dichloro-16β-methyl-1,4-pregnadiene-17α,21-diol-3,20-dione 21-acetate;
2) 9α,11β-dichloro-16α-methyl-1,4-pregnadiene-17α,21-diol-3,20-dione 17,21-di-n-butyrate;
3) 9α,11β-dichloro-1,4-pregnadiene-17α,21-diol-3,20-dione 21-acetate.

Isolate and purify each of the resultant products in a manner similar to that described in Example 1A, to obtain, respectively.

1) 16β-methyl-19-nor-pregna-1,3,5(10),6,8-pentaene-3,17α,21-triol-20-one 21-acetate; m.p. = 182—184°C; $[\alpha]_D^{26}$ = +122° (chloroform); $\lambda_{max}^{methanol}$ [in nm] = 229 ($\varepsilon$ = 67,000), 258 ($\varepsilon$ = 3,700), 268 ($\varepsilon$ = 4,800), 279 ($\varepsilon$ = 5,500), 291 ($\varepsilon$ = 4,000), 327 ($\varepsilon$ = 2,400), 340 ($\varepsilon$ = 2,700);

2) 16α-methyl-19-nor-pregna-1,3,5(10),6,8-pentaene-3,17α,21-triol-20-one 17,21-di-n-butyrate; m.p. = 200—202°C; $[\alpha]_D^{26}$ = −15° (dioxane); $\lambda_{max}^{methanol}$ [in nm] = 228 ($\varepsilon$ = 67,000), 257 ($\varepsilon$ = 4,000), 268 ($\varepsilon$ = 5,100), 279 ($\varepsilon$ = 5,700), 290 ($\varepsilon$ = 4,000), 325 ($\varepsilon$ = 2,300), 340 ($\varepsilon$ = 2,700);

3) 19-nor-pregna-1,3,5(10),6,8-pentaene-3,17α,21-triol-20-one 21-acetate; m.p. = 185—190°C, $[\alpha]_D^{26}$ = +91° (chloroform), $\lambda_{max}^{methanol}$ [in nm] = 229 ($\varepsilon$ = 66,400), 258 ($\varepsilon$ = 3,600), 268 ($\varepsilon$ = 4,900), 281 ($\varepsilon$ = 5,700), 291 ($\varepsilon$ = 4,200), 327 ($\varepsilon$ = 2,600), 340 ($\varepsilon$ = 3,100).

B. 19-Nor-pregna-1,3,5(10),6,8,14-hexaene-3,17α,21-triol-20-one Derivatives

In a manner similar to that described in Example 1B, treat each of the 19-nor-pregna-1,3,5(10),6,8-pentaenes obtainable from Example 2A with DDQ in dioxane and isolate and purify each of the resultant products in a manner similar to that described, to obtain, respectively,

1) 16β-methyl-19-nor-pregna-1,3,5(10),6,8,14-hexaene-3,17α,21-triol-20-one 21-acetate; m.p. = 177—179°C, $[\alpha]_D^{26}$ = +95° (chloroform); $\lambda_{max}^{methanol}$ [in nm] = 253 ($\varepsilon$ = 51,300), 262 ($\varepsilon$ = 51,800), 284 ($\varepsilon$ = 13,400), 295 ($\varepsilon$ = 18,100), 306 ($\varepsilon$ = 16,800, 327 ($\varepsilon$ = 2,800), 354 ($\varepsilon$ = 1,700);

2) 16α-methyl-19-nor-pregna-1,3,5(10),6,8,14-hexaene-3,17α,21-triol-20-one 17,21-di-n-butyrate; m.p. = 188—190°C, $[\alpha]_D^{26}$ = −201° (chloroform); $\lambda_{max}^{methanol}$ [in nm] = 246 ($\varepsilon$ = 38,700), 255 ($\varepsilon$ = 51,300), 264 ($\varepsilon$ = 51,300), 287 ($\varepsilon$ = 15,300), 297 ($\varepsilon$ = 20,000), 309 ($\varepsilon$ = 18,700), 339 ($\varepsilon$ = 2,000), 355 ($\varepsilon$ = 1,500);

3) 19-nor-pregna-1,3,5(10),6,8,14-hexaene-3,17α,21-triol-20-one 21-acetate; m.p. = 212—216°C, $[\alpha]_D^{26}$ = −30° (dioxane), $\lambda_{max}^{methanol}$ [in nm] = 245 ($\varepsilon$ = 38,000), 253 ($\varepsilon$ = 49,900), 262 ($\varepsilon$ = 46,700), 286 ($\varepsilon$ = 13,300), 295 ($\varepsilon$ = 17,900), 307 ($\varepsilon$ = 16,500), 338 ($\varepsilon$ = 2,000), 355 ($\varepsilon$ = 1,600).

EXAMPLE 3

3-ALKOXY DERIVATIVES OF 19-NOR-PREGNA-1,3,5(10),6,8,14-HEXAENE-3,17α,21-TRIOL-20-ONES

A. 3-Methoxy-16α-methyl-19-nor-pregna-1,3,5(10),6,8,14-hexaene-17α,21-diol-20-one 21-Acetate

To a solution of 16α-methyl-19-nor-pregna-1,3,5(10),6,8,14-hexaene-3,17α,21-triol-20-one 21-acetate (1 g) in ethyl acetate (50 ml), add a solution of diazomethane in ether (molar quantity of diazomethane being greater than that of pregnahexaene). Allow the reaction mixture to stand overnight at room temperature, then distill the excess diazomethane and ether. Purify the resultant residue via chromatography on silica gel preparative plates utilizing as solvent system chloroform:ethyl acetate (4:1). Remove the band containing the desired product (as visualized under ultraviolet light) by extraction with ethyl acetate. Evaporate the ethyl acetate and crystallize the resultant residue from petroleum ether/ether, to obtain 3-methoxy-16α-methyl-19-nor-pregna-1,3,5(10),6,8,14-hexaene-17α,21-diol-20-one 21-acetate; yield = 230 mg; m.p. = 186—188°C; $[\alpha]_D^{26} = -109°$ (chloroform); $\lambda_{max}^{methanol}$ [in nm] = 246 ($\varepsilon$ = 36,600), 254 ($\varepsilon$ = 46,300), 264 ($\varepsilon$ = 47,000), 283 ($\varepsilon$ = 14,700), 293 ($\varepsilon$ = 20,200), 306 ($\varepsilon$ = 19,700), 335 ($\varepsilon$ = 1,700), 352 ($\varepsilon$ = 1,300).

B. Other 3-Methoxy-19-nor-pregna-1,3,5(10),6,8,14-hexaene-17α,21-diol-20-ones

In similar manner, treat each of the 3-(free-hydroxy)-19-nor-pregna-1,3,5(10),6,8,14-hexaenes of Example 2B with diazomethane, to obtain, respectively,

1) 3-methoxy-16β-methyl-19-nor-pregna-1,3,5(10),6,8,14-hexaene-17α,21-diol-20-one 21-acetate;

2) 3-methoxy-16α-methyl-19-nor-pregna-1,3,5(10),6,8,14-hexaene-17α,21-diol-20-one 17,21-di-n-butyrate; and

3) 3-methoxy-19-nor-pregna-1,3,5(10),6,8,14-hexaene-17α,21-diol-20-one 21-acetate.

C. Other 3-Alkoxy Derivatives

Following the procedures of Examples 3A and 3B, but substituting for diazomethane other diazoalkane solutions, e.g. diazoethane, there are obtained the corresponding 3-alkoxy derivatives, e.g. the 3-ethoxy derivatives, corresponding to the 3-methoxy products of Examples 3A and 3B.

EXAMPLE 4

19-NOR-PREGNA-1,3,5(10),6,8,14-HEXAENE-3,17α,21-TRIOL-20-ONES

A. 16α-Methyl-19-nor-pregna-1,3,5(10),6,8,14-hexaene-3,17α,21-triol-20-one

To a solution of 16α-methyl-19-nor-pregna-1,3,5(10),6,8,14-hexaene-3,17α,21-triol-20-one 21-acetate (1 g) in methanol (70 ml) under an atmosphere of nitrogen, add aqueous sodium bicarbonate (10%, 5 ml). Heat at reflux temperature for 30 minutes, cool, add dilute acetic acid until the reaction mixture is at about pH 7, pour into water and extract with ethyl acetate. Wash the combined extracts with water, dry over magnesium sulfate, and evaporate. Crystallize the resultant residue from chloroform/ethyl acetate, to obtain 16α-methyl-19-nor-pregna-1,3,5(10),6,8,14-hexaene-3,17α,21-triol-20-one; yield = 697 mg; $[\alpha]_D^{26} = -188°$ (dioxane); m.p. = 220—225°C; $\lambda_{max}^{methanol}$ [in nm] = 246 ($\varepsilon$ = 35,000), 255 ($\varepsilon$ = 45,100), 264 ($\varepsilon$ = 45,900), 285 ($\varepsilon$ = 13,800), 296 ($\varepsilon$ = 18,500), 308 ($\varepsilon$ = 17,900), 338 ($\varepsilon$ = 2,600), 355 ($\varepsilon$ = 1,700).

B. Other 19-Nor-pregna-1,3,5(10),6,8,14-hexaene-3,17α,21-triol-20-ones

In similar manner, treat each of 16β-methyl-19-nor-pregna-1,3,5(10),6,8,14-hexaene-3,17α,21-triol-20-one 21-acetate and 19-nor-pregna-1,3,5(10),6,8,14-hexaene-3,17α,21-triol-20-one 21-acetate with aqueous sodium bicarbonate and isolate and purify the resultant products in the described manner, to obtain, respectively, 16β-methyl-19-nor-pregna-1,3,5(10),6,8,14-hexaene-3,17α,21-triol-20-one and 19-nor-pregna-1,3,5(10),6,8,14-hexaene-3,17α,21-triol-20-one.

EXAMPLE 5

17-MONO-LOWER ALKANOATES AND 21-MONO-LOWER ALKANOATES FROM THE CORRESPONDING 19-NOR-PREGNA-1,3,5(10),6,8,14-HEXAENE-3,17α,21-TRIOL-20-ONES

A. 21-Propionate and 17-Propionate of 16α-Methyl-19-nor-pregna-1,3,5(10),6,8,14-hexaene-3,17α,21-triol-20-one

(1) To a solution of 16α-methyl-19-nor-pregna-1,3,5(10),6,8,14-hexaene-3,17α,21-triol-20-one (697 mg) in dimethylsulfoxide (9.7 ml), add triethyl orthopropionate (0.97 ml) and p-toluenesulfonic acid (97 mg). Stir the reaction mixture at room temperature for 5 hours, then add acetic acid/water (14 ml, 9:1) and stir the mixture at room temperature overnight. Pour the reaction mixture into water, separate the resultant precipitate by filtration and wash it with water, then chromatograph the precipitate over silica gel, eluting with methylene-chloride/ether (19:1). Combine

the like early fractions as determined by thin-layer chromatography, evaporate, crystallize the resultant residue from ether, and filter to obtain 16α-methyl-19-nor-pregna-1,3,5(10),6,8,14-hexaene-3,17α,21-triol-20-one 21-propionate; yield = 138 mg; m.p. = 218—222°C; $[\alpha]_D^{26} = -109°$ (chloroform); $\lambda_{max}^{methanol}$ [in nm] = 246 (ε = 36,500), 255 (ε = 47,400), 264 (ε = 48,200), 286 (ε = 13,900), 296 (ε = 19,300), 309 (ε = 18,200), 338 (ε = 2,000), 355 (ε = 1,500).

(2) Continue eluting with the same solvent and combine the like later fractions as determined by thin-layer chromatography, evaporate, crystallize the resultant precipitate from ether/petroleum ether and filter, to obtain 16α-methyl-19-nor-pregna-1,3,5(10),6,8,14-hexaene-3,17α,21-triol-20-one 17 propionate; yield = 41 mg; m.p. = 115—120°C; $[\alpha]_D^{26} = -212°$ (chloroform).

B. 21- and 17-Monoesters of Other 19-Nor-pregna-1,3,5(10), 6,8,14-hexaene-3,17α,21-triol-20-ones

In similar manner, treat each of 16β-methyl-19-nor-pregna-1,3,5(10),6,8,14-hexaene-3,17α,21-triol-20-one and 19-nor-pregna-1,3,5(10),6,8,14-hexaene-3,17α,21-triol-20-one with triethyl orthopropionate and p-toluene-sulfonic acid, followed by treatment with aqueous acetic acid, and isolate and purify each of the resultant products in a manner similar to that described hereinabove, to obtain, respectively, 16β-methyl-19-nor-pregna-1,3,5(10),6,8,14-hexaene-3,17α,21-triol-20-one 21-propionate and 16β-methyl-19-nor-pregna-1,3,5(10),6,8,14-hexaene-3,17α,21-triol-20-one 17-propionate, 19-nor-pregna-1,3,5(10),6,8,14-hexaene-3,17α, 21-triol-20-one 21-propionate and 19-nor-pregna-1,3,5(10),6,8,14-hexaene-3,17α,21-triol-20-one 17-propionate.

EXAMPLE 6

15-CHLORO-19-NOR-PREGNA-1,3,5(10),6,8,14-HEXAENE-3,17α,21-TRIOL-20-ONES

A. 15-Chloro-16α-methyl-19-nor-pregna-1,3,5(10),6,8,14-hexaene-3,17α,21-triol-20-one 21-Acetate

To a saturated solution of hydrogen chloride gas in dioxane (50 ml), add 16α-methyl-19-nor-pregna-1,3,5(10),6,8-pentaene-3,17α,21-triol-20-one 21-acetate (382 mg), warm slightly to dissolve. To the resulting solution add 2,3-dichloro-5,6-dicyanobenzoquinone (454 mg) and stir the reaction mixture at room temperature for 30 minutes. Evaporate the dioxane at room temperature for 30 minutes. Evaporate the dioxane, dissolve the resultant residue in ether and percolate the ether solution through an alumina column. Evaporate the combined eluates and chromatograph the resultant residue over silica gel eluting with petroleum ether/ether gradient elution. Combine the like fractions containing the desired product as determined by thin-layer chromatography and evaporate the combined eluates. Recrystallize the resultant residue from ether/petroleum ether and filter the resultant precipitate, to give 15-chloro-16α-methyl-19-nor-pregna-1,3,5(10),6,8,14-hexaene-3,17α,21-triol-20-one 21-acetate; yield = 131 mg; m.p. = 225—258°C; $[\alpha]_D^{26} = -185°$ (chloroform); $\lambda_{max}^{methanol}$ [in nm] = 258 (ε = 44,700), 266 (ε = 49,200), 290 (ε = 12,500), 303 (ε = 15,000), 315 (ε = 14,600), 336 (ε = 3,900), 354 (ε = 2,900).

B. Other 15-Chloro-19-nor-pregna-1,3,5(10),6,8,14-hexaene-3,17α,21-triol-20-ones

In similar manner, treat each of the 19-nor-pregnapentaenes obtainable from Example 2A with DDQ and hydrogen chloride, and isolate and purify each of the resultant products in a manner similar to that described hereinabove, to obtain, respectively,

1) 15-chloro-16β-methyl-19-nor-pregna-1,3,5(10),6,8,14-hexaene-3,17α,21-triol-20-one 21-acetate;

2) 15-chloro-16α-methyl-19-nor-pregna-1,3,5(10),6,8,14-hexaene-3,17α,21-triol-20-one 17,21-di-in-butyrate;

3) 15-chloro-19-nor-pregna-1,3,5(10),6,8,14-hexaene-3,17α,21-triol-20-one 21-acetate.

EXAMPLE 7

PREPARATION OF 3-CARBOXYLATE ESTERS

A. 16α-Methyl-19-nor-pregna-1,3,5(10),6,8,14-hexaene-3,17α,21-triol-20-one 3,21-Diacetate

To a solution of 16α-methyl-19-nor-pregna-1,3,5(10),6,8,14-hexaene-3,17α,21-triol-20-one 21-acetate (450 mg) in pyridine (2 ml), add acetic anhydride (1 ml) and allow the reaction mixture to stand at room temperature overnight. Pour the reaction mixture into dilute hydrochloric acid, separate the resultant precipitate by filtration, wash it with water, dry and crystallize from ether, to obtain 16α-methyl-19-nor-pregna-1,3,5(10),6,8,14-hexaene-3,17α,21-triol-20-one 3,21-diacetate; yield = 232 mg; m.p. = 153—157°C, $[\alpha]_d^{26} = -93°$ (chloroform); $\lambda_{max}^{methanol}$ [in nm] = 388 (ε = 29,300), 246 (ε = 32,600), 255 (ε = 41,360), 264 (ε = 41,600), 283 (ε = 14,500), 293 (ε = 18,000), 306 (ε = 16,700), 330 (ε = 600).

B. 16$\alpha$-Methyl-19-nor-pregna-1,3,5(10),6,8,14-hexaene-3,17$\alpha$,21-triol-20-one 3-Benzoate 21-Acetate

In the procedure of Example 7A, by substituting for acetic anhydride an equivalent quantity of benzoyl chloride, there is obtained 16$\alpha$-methyl-19-nor-pregna-1,3,5(10),6,8,14-hexaene-3,17$\alpha$,21-triol-20-one 3-benzoate 21-acetate; m.p. = 197—202°C; $[\alpha]_D^{26} = -68°$ (chloroform); $\lambda_{max}^{methanol}$ [in nm] = 238 ($\varepsilon$ = 39,300), 256 ($\varepsilon$ = 44,800), 265 ($\varepsilon$ = 45,500), 283 ($\varepsilon$ = 16,000), 295 ($\varepsilon$ = 17,900), 307 ($\varepsilon$ = 17,000).

C. 3-Carboxylate Esters of Other 19-Nor-pregna-1,3,5(10),6,8,14-hexaene Derivatives

(1) In similar manner treat each of the 3-(free-hydroxy)- or 3,21-di-(free-hydroxy)-19-nor-pregna-1,3,5(10),6,8,14-hexaene compounds obtainable from Examples 2B, 5 and 6 (possibly containing a further free hydroxy group in position 17) with acetic anhydride in pyridine or benzoyl chloride in pyridine, to obtain the corresponding 3-acetate or 3-benzoate ester thereof, or the corresponding 3,21-diacetate or 3,21-dibenzoate respectively.

(2) Treat each of the 3,17$\alpha$,21-tri-(free-hydroxy)-19-nor-pregna-1,3,5(10),6,8,14-hexaenes prepared in Example 4 with acetic anhydride in pyridine or benzoyl chloride in pyridine according to procedures of above Examples 7A and 7B, to obtain, respectively,

1) 16$\alpha$-methyl-19-nor-pregna-1,3,5(10),6,8,14-hexaene-3,17$\alpha$,21-triol-20-one 3,21-diacetate;
2) 16$\alpha$-methyl-19-nor-pregna-1,3,5(10),6,8,14-hexaene-3,17$\alpha$,21-triol-20-one 3,21-dibenzoate;
3) 16$\beta$-methyl-19-nor-pregna-1,3,5(10),6,8,14-hexaene-3,17$\alpha$,21-triol-20-one 3,21-diacetate;
4) 16$\beta$-methyl-19-nor-pregna-1,3,5(10),6,8,14-hexaene-3,17$\alpha$,21-triol-20-one 3,21-dibenzoate;
5) 19-nor-pregna-1,3,5(10),6,8,14-hexaene-3,17$\alpha$,21-triol-20-one 3,21-diacetate;
6) 19-nor-pregna-1,3,5(10),6,8,14-hexaene-3,17$\alpha$,21-triol-20-one 3,21-dibenzoate.

## EXAMPLE 8

16$\alpha$-METHYL-19-NOR-PREGNA-1,3,5(10),6,8,14-HEXAENE-3,17$\alpha$,21-TRIOL-11,20-DIONE 3-BENZOATE 21-ACETATE

A. 16$\alpha$-Methyl-19-nor-pregna-1,3,5(10),6,8-pentaene-3,17$\alpha$,21-triol-11,20-dione 3-Benzoate 21-Acetate

To a solution of 9$\alpha$-bromo-16$\alpha$-methyl-1,4-pregnadiene-17$\alpha$,21-diol-3,11,20-trione 21-acetate (30 g) in pyridine (240 ml) add benzoyl chloride (60 ml) and heat the reaction mixture at 60°C for 20 hours, cool and pour into dilute hydrochloric acid. Extract the aqueous solution with ethyl acetate, wash the combined extracts with water, and evaporate. Chromatograph the resultant residue over silica gel eluting with petroleum ether/ether gradient. Combine the like fractions containing the desired product as determined by thin-layer chromatography, evaporate, then crystallize the resultant residue from ether, to obtain 16$\alpha$-methyl-19-nor-pregna-1,3,5(10),6,8-pentaene-3,17$\alpha$,21-triol-11,20-dione 3-benzoate 21-acetate (yield 13.1 g); m.p. = 183—184°C; $[\alpha]_D^{26} = +63°$ (dioxane); $\lambda_{max}^{methanol}$ [in nm] = 215 ($\varepsilon$ = 40,600), 237 ($\varepsilon$ = 38,400), 314 ($\varepsilon$ = 7,900).

B. 16$\alpha$-Methyl-19-nor-pregna-1,3,5(10),6,8,14-hexaene-3,17$\alpha$,21-triol-11,20-dione 3-Benzoate 21-Acetate

To a solution of 16$\alpha$-methyl-19-nor-pregna-1,3,5(10),6,8-pentaene-3,17$\alpha$,21-triol-11,20-dione 3-benzoate 21-acetate (10.4 g) in dioxane (500 ml) add DDQ (13.33 g = 2.4 equivalents) and heat the reaction mixture at reflux temperature for 48 hours. Then evaporate *in vacuo*, dissolve the resultant residue in methylene chloride and percolate through alumina ("activity V", i.e. anhydrous alumina the activity of which has been modified by addition of 15% water). Evaporate the combined eluates to a small volume and chromatograph over silica gel, eluting with petroleum ether/ether gradient. Combine the like fractions containing the desired product as determined by thin-layer chromatography, and evaporate, then recrystallize the resultant residue from ether, to obtain 16$\alpha$-methyl-19-nor-pregna-1,3,5(10),6,8,14-hexaene-3,17$\alpha$,21-triol-11,20-dione 3-benzoate 21-acetate; m.p. 144—145°C; $[\alpha]_D^{26} = +1°$ (dioxane); $\lambda_{max}^{methanol}$ [in nm] = 234 ($\varepsilon$ = 37,700), 270 ($\varepsilon$ = 42,900), 277 (shoulder) ($\varepsilon$ = 40,400), 312 ($\varepsilon$ = 9,000), 349 ($\varepsilon$ = 6,000) 365 ($\varepsilon$ = 5,300).

## EXAMPLE 9

16$\alpha$-METHYL-19-NOR-PREGNA-1,3,5(10),6,8,14-HEXAENE-3,17$\alpha$,21-TRIOL-11,20-DIONE

By subjecting 16$\alpha$-methyl-19-nor-pregna-1,3,5(10),6,8,14-hexaene-3,17$\alpha$,21-triol-11,20-dione 3-benzoate 21-acetate to substantially the conditions of Example 4A, there is obtained 16$\alpha$-methyl-19-nor-pregna-1,3,5(10),6,8,14-hexaene-3,17$\alpha$,21-triol-11,20-dione; m.p. = 163—165°C; $[\alpha]_D^{26}$ = −107° (dioxane); $\lambda_{max}^{methanol}$ [in nm] = 236 ($\varepsilon$ = 24,100), 276 ($\varepsilon$ = 34,000), 317 ($\varepsilon$ = 5,600), 378 ($\varepsilon$ = 5,700).

EXAMPLE 10

OTHER ESTER DERIVATIVES OF 16$\alpha$-METHYL-19-NOR-PREGNA-1,3,5(10),6,8,14-HEXAENE-3,17$\alpha$,21-TRIOL-11,20-DIONE

A. The 21-Propionate and the 17-Propionate of 16$\alpha$-Methyl-19-nor-pregna-1,3,5(10),6,8,14-hexaene-3,17$\alpha$,21-triol-11,20 dione

By subjecting 16$\alpha$-methyl-19-nor-pregna-1,3,5(10),6,8,14-hexaene-3,17$\alpha$,21-triol-11,20-dione (100 mg) to substantially the conditions of Example 5A, there are obtained respectively, 16$\alpha$-methyl-19-nor-pregna-1,3,5(10),6,8,14-hexaene-3,17$\alpha$,21-triol-11,20-dione 21-propionate (25 mg; the residue from the least polar band), and 16$\alpha$-methyl-19-nor-pregna-1,3,5(10),6,8,14-hexaene-3,17$\alpha$,21-triol-11,20-dione 17-propionate (25 mg; the residue from the most polar band).

B. 16$\alpha$-Methyl-19-nor-pregna-1,3,5(10),6,8,14-hexaene-3,17$\alpha$,21-triol-11,20-dione 3,17,21-Tripropionate

To a suspension of 16$\alpha$-methyl-19-nor-pregna-1,3,5(10),6,8,14-hexaene-3,17$\alpha$,21-triol-11,20-dione (2 g) in propionic acid (20 ml) containing *p*-toluenesulfonic acid (200 mg) at −5°C, add dropwise over a 40-minute period trifluoroacetic anhydride (8 ml). Allow the reaction mixture to warm to room temperature, then stir for 24 hours. Pour the reaction mixture onto ice/water and extract with ethyl acetate. Wash the combined extracts with aqueous sodium bicarbonate, then with water and evaporate *in vacuo*. Chromatograph the resultant residue over silica gel eluting with petroleum ether/ether gradient. Combine the like fractions containing the desired product as determined by thin-layer chromatography, to obtain 16$\alpha$-methyl-19-nor-pregna-1,3,5(10),6,8,14-hexaene-3,17$\alpha$,21-triol-11,20-dione 3,17,21-tripropionate; yield 2.04 g; $[\alpha]_D^{26} = -82°$ (dioxane); $\lambda_{max}^{methanol}$ [in nm] = 233 ($\varepsilon$ = 26,300), 260 (shoulder) ($\varepsilon$ = 30,700), 269 ($\varepsilon$ = 36,100), 278 ($\varepsilon$ = 34,800), 316 ($\varepsilon$ = 7,700), 345 (shoulder) ($\varepsilon$ = 5,400), 364 ($\varepsilon$ = 4,600).

C. 16$\alpha$-Methyl-19-nor-pregna-1,3,5(10),6,8,14-hexaene-3,17$\alpha$,21-triol-11,20-dione 3,21-Diacetate

Subject 16$\alpha$-methyl-19-nor-pregna-1,3,5(10),6,8,14-hexaene-3,17$\alpha$,21-triol-11,20-dione to substantially the conditions of Example 7A, to obtain 16$\alpha$-methyl-19-nor-pregna-1,3,5(10),6,8,14-hexaene-3,17$\alpha$,21-triol-11,20-dione 3,21-diacetate; $[\alpha]_D^{26} = -1'$ (dioxane); $\lambda_{max}^{methanol}$ [in nm] = 234 ($\varepsilon$ = 25,900), 269 ($\varepsilon$ = 33,300), 278 ($\varepsilon$ = 29,300), 315 ($\varepsilon$ = 7,800), 348 ($\varepsilon$ = 5,300), 365 ($\varepsilon$ = 4,700).

D. 16$\alpha$-Methyl-19-nor-pregna-1,3,5(10),6,8,14-hexaene-3,17$\alpha$,21-triol-11,20-dione 3,21-Dipropionate

Following the procedure of Example 10C, by utilizing an equivalent quantity of propionic anhydride instead of acetic anhydride, there is obtained 16$\alpha$-methyl-19-nor-pregna-1,3,5(10),6,8,14-hexaene-3,17$\alpha$,21-triol-11,20-dione 3,21-dipropionate; m.p. = 135—137°C; $[\alpha]_D^{26} = +2°$ (dioxane); $\lambda_{max}^{methanol}$ [in nm] = 233 ($\varepsilon$ = 24,000), 269 ($\varepsilon$ = 32,700), 279 (shoulder) ($\varepsilon$ = 28,500), 315 ($\varepsilon$ = 7,100), 349 ($\varepsilon$ = 5,000), 365 ($\varepsilon$ = 4,300).

EXAMPLE 11

16$\alpha$ - METHYL - 19 - NOR - PREGNA - 1,3,5(10),6,8,14 - HEXAENE - 3,11$\beta$,17$\alpha$,21 - TETROL - 20 - ONE 3,17,21 - TRIPROPIONATE AND 17,21 - DIPROPIONATE

To a solution of 16$\alpha$-methyl-19-nor-pregna-1,3,5(10),6,8,14-hexaene-3,17$\alpha$,21-triol-11,20-dione 3,17,21-tripropionate (1 g) in tetrahydrofuran/methanol (50 ml 1:1), dried over an alumina column at 0°C, add sodium borohydride (220 mg = 3 equivalents) portionwise over a 5-minute period. Stir the reaction mixture for an additional 10 minutes, then bring to neutrality by adding glacial acetic acid dropwise. Pour the reaction mixture into water, extract with ethyl acetate, wash the combined extracts with water and evaporate. Chromatograph the resultant residue over silica gel GF column, eluting with chloroform/ethyl acetate (9:1). Combine the like fractions as determined by thin-layer chromatography, and evaporate each of the three different combined fractions to residues comprising, respectively,

1) 16$\alpha$ - methyl - 19 - nor - pregna - 1,3,5(10),6,8,14 - hexaene - 3,11$\alpha$,17$\alpha$,21 - tetrol - 20 - one 3,17$\alpha$,21 - tripropionate (yield: 53 mg);

2) 16$\alpha$ - methyl - 19 - nor - pregna - 1,3,5(10),6,8,14 - hexaene - 3,11$\beta$,17$\alpha$,21 - tetrol - 20 - one 3,17$\alpha$,21 - tripropionate (yield: 502 mg). After purification by recrystallization from petroleum ether/ether: $[\alpha]_D^{26} = -136°$ (dioxane); $\lambda_{max}^{methanol}$ [in nm] = 237 ($\varepsilon$ = 30,200), 244 ($\varepsilon$ = 34,000), 253 ($\varepsilon$ = 45,100), 262 ($\varepsilon$ = 44,200), 281 ($\varepsilon$ = 16,200), 291 ($\varepsilon$ = 20,300), 304 ($\varepsilon$ = 18,500), 328 ($\varepsilon$ = 900), 344 ($\varepsilon$ = 500);

3) 16$\alpha$-methyl-19-nor-pregna-1,3,5(10),6,8,14-hexaene-3,11$\beta$, 17$\alpha$,21-tetrol-20-one 17,21-dipropionate (yield: 93 mg). After purification by recrystallization from petroleum ether/ether: $[\alpha]_D^{26} = -145°$ (dioxane); $\lambda_{max}^{methanol}$ [in nm] = 236 (shoulder) ($\varepsilon$ = 28,100), 244 ($\varepsilon$ = 33,800), 256 ($\varepsilon$ = 40,200), 265 ($\varepsilon$ = 40,200), 287 (shoulder) ($\varepsilon$ = 12,600), 297 ($\varepsilon$ = 16,400), 308 ($\varepsilon$ = 15,500),

337 ($\varepsilon$ = 2,700).

EXAMPLE 12

6 - FLUORO - 16$\alpha$ - METHYL - 19 - NOR - PREGNA - 1,3,5(10),6,8,14 - HEXAENE - 3,17$\alpha$,21 - TRIOL - 20 - ONE 21 - ACETATE

A. 6-Fluoro-16$\alpha$-methyl-19-nor-pregna-1,3,5(10),6,8,-pentaene-3,17$\alpha$,21-triol-20-one 21-Acetate

Add 6$\alpha$-fluoro-9$\alpha$,11$\beta$-dichloro-16$\alpha$-methyl-1,4-pregnadiene-17$\alpha$,21-diol-3,20-dione 21-acetate (4.2 g) to refluxing dimethylformamide (200 ml) and continue heating at reflux temperature for 30 minutes. Pour the reaction mixture into saturated aqueous sodium chloride solution, separate the resultant precipitate by filtration. Dissolve the precipitate in ethyl acetate, and fractionally crystallize to obtain both, 6$\alpha$-fluoro-16$\alpha$-methyl-pregna-1,3,8(14),9(11)-tetraene-17$\alpha$,21-diol-3,20-dione 21-acetate and 6-fluoro-16$\alpha$-methyl-19-nor-pregna-1,3,5(10),6,8-pentaene-3,17$\alpha$,21-triol-20-one 21-acetate. Further purify the latter compound by crystallization from methylene chloride, yield: 316 mg; $[\alpha]_D^{26}$ = +88.0° (dioxane); m.p. = 238—241°C; $\lambda_{max}^{methanol}$ [in nm] = 238 ($\varepsilon$ = 50,500), 270 ($\varepsilon$ = 5,000), 281 ($\varepsilon$ = 5,000), 293 ($\varepsilon$ = 3,400), 315 (shoulder) ($\varepsilon$ = 1,700), 330 ($\varepsilon$ = 2,400), 344 ($\varepsilon$ = 2,600).

B. 6-Fluoro-16$\alpha$-methyl-19-nor-pregna-1,3,5(10),6,8,14-hexaene-3,17$\alpha$,21-triol-20-one 21-Acetate

Subject 6-fluoro-16$\alpha$-methyl-19-nor-pregna-1,3,5(10),6,8-pentaene-3,17$\alpha$,21-triol-20-one 21-acetate (200 mg) to substantially the conditions of Example 1B, to obtain 6-fluoro-16$\alpha$-methyl-19-nor-pregna-1,3,5(10),6,8,14-hexaene-3,17$\alpha$,21-triol-20-one 21-acetate, yield: 35 mg; m.p. = 200—202°C; $\lambda_{max}^{methanol}$ [in nm] = 248 (shoulder) ($\varepsilon$ = 36,300), 255 ($\varepsilon$ = 48,100), 265 ($\varepsilon$ = 49,200), 288 ($\varepsilon$ = 13,900), 299 ($\varepsilon$ = 19,500), 311 ($\varepsilon$ = 18,500), 342 ($\varepsilon$ = 2,100), 359 ($\varepsilon$ = 1,500).

EXAMPLE 13

16$\beta$-METHYL-19-NOR-PREGNA-1,3,5(10),6,8,14-HEXAENE-3,11$\beta$,17$\alpha$,21-TETROL-20-ONE 21-ACETATE

A. 16$\beta$-Methyl-pregna-1,4,6,8-tetraene-11$\beta$,17$\alpha$,21-triol-3,20-dione 21-Acetate

To a mixture of 9$\alpha$-chloro-16$\beta$-methyl-pregna-1,4,6-triene-11$\beta$,17$\alpha$,21-triol-3,20-dione 21-acetate (3.44 g) in acetone (700 ml) add potassium acetate (10.3 g) and reflux the reaction mixture with stirring for 48 hours. Filter the reaction mixture, evaporate the filtrate *in vacuo* to a low volume, pour into water and extract the aqueous mixture with ethyl acetate. Wash the combined organic extracts with water and evaporate to a volume of about 100 ml. Separate the resultant crystalline solid by filtration, and dry, to obtain 16$\beta$-methyl-pregna-1,4,6,8-tetraene-11$\beta$,17$\alpha$,21-triol-3,20-dione 21-acetate, yield: 1.96 g; m.p. = 175—180°C; $[\alpha]_D^{26}$ = +786° (pyridine); $\lambda_{max}^{methanol}$ [in nm] = 230 (shoulder) ($\varepsilon$ = 10,600), 264 ($\varepsilon$ = 10,000), 388 ($\varepsilon$ = 6,500).

B. 16$\beta$-Methyl-19-nor-pregna-1,3,5(10),6,8,-pentaene-3,11$\beta$,17$\alpha$,21-tetrol-20-one 21-Acetate

To a solution of 16$\beta$-methyl-pregna-1,4,6,8-tetraene-11$\beta$,17$\alpha$,21-triol-3,20-dione 21-acetate (850 mg) in tetrahydrofuran (200 ml) add 1 N hydrochloric acid (20 ml). Stir the reaction mixture at room temperature for 1 hour, then pour the reaction mixture into 1 liter of saturated aqueous sodium chloride solution and extract with ethyl acetate. Wash the combined ethyl acetate extracts with water and evaporate to a volume of about 25 ml. Separate the resultant crystalline precipitate by filtration and dry, to obtain 16$\beta$-methyl-19-nor-pregna-1,3,5(10),6,8-pentaene-3,11$\beta$,17$\alpha$,21-tetrol-20-one 21-acetate, yield: 368 mg; m.p. = 203—207°C; $[\alpha]_D^{26}$ = +172° (pyridine); $\lambda_{max}^{methanol}$ [in nm] = 233 ($\varepsilon$ = 69,100), 267 ($\varepsilon$ = 4,800), 278 ($\varepsilon$ = 5,400), 315 ($\varepsilon$ = 1,900), 327 ($\varepsilon$ = 2,300) 340 ($\varepsilon$ = 2,700).

C. 16$\beta$-Methyl-19-nor-pregna-1,3,5(10),6,8,14-hexaene-3,11$\beta$,17$\alpha$,21-tetrol-20-one 21-Acetate

Subject 16$\beta$-methyl-19-nor-pregna-1,3,5(10),6,8-pentaene-3,11$\beta$,17$\alpha$,21-tetrol-20-one 21-acetate (199 mg) to substantially the conditions of Example 1B, to obtain 16$\beta$-methyl-19-nor-pregna-1,3,5(10),6,8,14-hexaene-3,11$\beta$,17$\alpha$,21-tetrol-20-one 21-acetate, yield: 91 mg; m.p. = 185—195°C; $\lambda_{max}^{methanol}$ [in nm] = 244 ($\varepsilon$ = 35,100), 254 ($\varepsilon$ = 44,000), 263 ($\varepsilon$ = 44,000), 285 (shoulder) ($\varepsilon$ = 12,400), 295 ($\varepsilon$ = 16,000), 306 ($\varepsilon$ = 14,900), 337 ($\varepsilon$ = 2,600), 354 ($\varepsilon$ = 2,200).

EXAMPLE 14

16$\beta$-METHYL-19-NOR-PREGNA-1,3,5(10),6,8,14-HEXAENE-3,17$\alpha$,21-TRIOL-11,20-DIONE 21-ACETATE

A. 16$\beta$-Methyl-pregna-1,4,6,8-tetraene-17$\alpha$,21-diol-3,11,20-trione 21-Acetate

To a solution of 16$\beta$-methyl-pregna-1,4,6,8-tetraene-11$\beta$,17$\alpha$,21-triol-3,20-dione 21-acetate (500 mg) in methylene chloride (50 ml) add finely powdered manganese dioxide (5 g), and stir at room temperature for 20 hours. Separate the manganese dioxide by filtration and wash with methylene chloride. Evaporate the combined filtrate and methylene chloride washings and crystallize the resultant

residue from ether, to yield 16β-methyl-pregna-1,4,6,8-tetraene-17α,21-diol-3,11,20-trione 21-acetate; m.p. = 185—188°C; $[\alpha]_D^{26}$ = +1164° (chloroform).

B. 16β-Methyl-19-nor-pregna-1,3,5(10),6,8-pentaene-3,17α,21-triol-11,20-dione 21-Acetate

Subject 16β-methyl-pregna-1,4,6,8-tetraene-17α,21-diol-3,11,20-trione 21-acetate to substantially the conditions of Example 13B, to obtain 16β-methyl-19-nor-pregna-1,3,5(10),6,8-pentaene-3,17α,21-triol-11,20-dione 21-acetate.

C. 16β-Methyl-19-nor-pregna-1,3,5(10),6,8,14-hexaene-3,17α,21-triol-11,20-dione 21-Acetate

In a manner similar to that described in Example 13C, treat 16β-methyl-19-nor-pregna-1,3,5(10),6,8-pentaene-3,17α,21-triol-11,20-dione 21-acetate with DDQ in dioxane and isolate the resultant product in a manner similar to that described, to obtain 16β-methyl-19-nor-pregna-1,3,5(10),6,8,14-hexaene-3,17α,21-triol-11,20-dione 21-acetate.

EXAMPLE 15

16α-METHYL-19-NOR-PREGNA-1,3,5(10),6,8,14-HEXAENE-3,11β,17α,21-TETROL-20-ONE

A. 16α-Methyl-17α,20; 20,21-bismethylenedioxy-19-nor-pregna-1,3,5(10),6,8,14-hexaene-3-ol-11-one

To a solution of 16α-methyl-19-nor-pregna-1,3,5(10),6,8,14-hexaene-3,17α,21-triol-11,20-dione (4.3 g) in methylene chloride (200 ml) under an atmosphere of nitrogen, add formaldehyde (200 ml, 37% aqueous solution) and concentrated hydrochloric acid (200 ml). Stir the mixture at room temperature for 4 hours, separate the two layers, extract the aqueous layer with methylene chloride, combine the organic layer and the methylene chloride extracts and wash with aqueous sodium bicarbonate, then with water. Evaporate the organic solution and chromatograph the resultant residue over silica gel, eluting with a petroleum ether/ether gradient. Combine the like fractions containing the desired product as determined by thin-layer chromatography and evaporate and crystallize the resultant residue from ether, to obtain 16α-methyl-17α,20; 20,21-bismethylenedioxy-19-nor-pregna-1,3,5(10),6,8,14-hexaene-3-ol-11-one.

B. 16α-Methyl-17α,20; 20,21-bismethylenedioxy-19-nor-pregna-1,3,5(10),6,8,14-hexaene-3,11β-diol

Subject 16α-methyl-17α,20; 20,21-bismethylenedioxy-19-nor-pregna-1,3,5(10),6,8,14-hexaene-3-ol-11-one to substantially the conditions of Example 11, to obtain 16α-methyl-17α,20; 20,21-bismethylenedioxy-19-nor-pregna-1,3,5(10),6,8,14-hexaene-3,11α-diol (from the combined early fractions) and 16α-methyl-17α,20; 20,21-bismethylenedioxy-19-nor-pregna-1,3,5(10),6,8,14-hexaene-3,11β-diol (from the combined like later fractions). Purify by crystallization from ether.

C. 16α-Methyl-19-nor-pregna-1,3,5(10),6,8,14-hexaene-3,11β,17α,21-tetrol 20-one

Add a suspension of 16α-methyl-17α,20; 20,21-bismethylenedioxy-19-nor-pregna-1,3,5(10),6,8,14-hexaene-3,11β-diol (990 mg = 2.5 mmol) to aqueous 45% hydrochloric acid (2.5 ml) at 0°C and stir the resulting suspension at 0°C for 1.5 hours. Bring the reaction mixture to neutrality by adding aqueous 5% potassium bicarbonate, then extract with ethyl acetate, wash the combined extracts with water and evaporate to a small volume. Separate the resultant crystals by filtration and dry, to obtain 16α-methyl-19-nor-pregna-1,3,5(10),6,8,14-hexaene-3,11β,17α,21-tetrol 20-one.

EXAMPLE 16

ALTERNATE PREPARATION OF 16α-METHYL-19-NOR-PREGNA-1,3,5(10),6,8,14-HEXAENE-3,11β,17α,21-TETROL-20-ONE

To a solution of 16α-methyl-19-nor-pregna-1,3,5(10),6,8,14-hexaene-3,11β,17α,21-tetrol-20-one 3,17α,21-tripropionate (271 mg) in methanol (25 ml), add sodium bicarbonate (3.0 ml, 5% aqueous solution) and stir overnight at room temperature. Add dilute hydrochloric acid until the reaction mixture is at about pH 7, then remove the methanol *in vacuo.* Add water to the resultant residue and extract with ether. Wash the combined ether extracts with water, dry over magnesium sulfate and evaporate. Crystallize the resultant residue from methylene chloride/ether to obtain 16α-methyl-19-nor-pregna-1,3,5(10),6,8,14-hexaene-3,11β,17α,21-tetrol-20-one; yield: 44 mg; m.p. = 160—163°C; $[\alpha]_D^{26}$ = −161° (dioxane), $\lambda_{max}^{methanol}$ [in nm] = 263 (shoulder) ($\varepsilon$ = 24,600), 245 ($\varepsilon$ = 31,000), 255 ($\varepsilon$ = 38,000), 264 ($\varepsilon$ = 38,700), 286 (shoulder) ($\varepsilon$ = 12,800), 296 ($\varepsilon$ = 16,200), 308 ($\varepsilon$ = 15,100), 338 ($\varepsilon$ = 2,700).

EXAMPLE 17

OTHER 19-NOR-PREGNA-1,3,5(10),6,8,14-HEXAENE-3,17α,21-TRIOL-20-ONES

A. Other 19-Nor-pregna-1,3,5(10),6,8-pentaene-3,17α,21-triol-20-ones

In a manner similar to that described in Example 1A, treat each of the following 9α,11β-dihalogeno-1,4-pregnadienes with lithium chloride in dimethylformamide.

1) 6α,16α-dimethyl-9α,11β-dichloro-1,4-pregnadiene-17α,21-diol-3,20-dione 21-acetate;
2) 6α-fluoro-9α,11β-dichloro-1,4-pregnadiene-17α,21-diol-3,20-dione 21-acetate;
3) 6α-fluoromethyl-9α,11β-dichloro-1,4-pregnadiene-17α,21-diol-3,20-dione 21-acetate;
4) 6α-difluoromethyl-9α,11β-dichloro-1,4-pregnadiene-17α,21-diol-3,20-dione 21-acetate;
5) 6α-trifluoromethyl-9α,11β-difluoro-1,4-pregnadiene-17α,21-diol-3,20-dione 21-acetate;
6) 9α,11β-dichloro-1,4-pregnadiene-16α,17α,21-triol-3,20-dione 16,21-diacetate;
7) 6α-fluoro-9α,11β-dichloro-16α,17α-isopropylidenedioxy-1,4-pregnadiene-21-ol-3,20-dione 21-acetate;
8) 6α-fluoro-9α,11β-dichloro-16α-methyl-1,4-pregnadiene-17α,21-diol-3,20-dione 21-acetate;
9) 6α-methyl-9α,11β-dichloro-1,4-pregnadiene-17α,21-diol-3,20-dione 21-acetate;
10) 6α,16β-dimethyl-9α,11β-dichloro-1,4-pregnadiene-17α,21-diol-3,20-dione 21-acetate.

Isolate and purify each of the resultant products in a manner similar to that described, to obtain, respectively,

1) 6,16α-dimethyl-19-nor-pregna-1,3,5(10),6,8-pentaene-3,17α,21-triol-20-one 21-acetate;
2) 6-fluoro-19-nor-pregna-1,3,5(10),6,8-pentaene-3,17α,21-triol-20-one 21-acetate;
3) 6-fluoromethyl-19-nor-pregna-1,3,5(10),6,8-pentaene-3,17α,21-triol-20-one 21-acetate;
4) 6-difluoromethyl-19-nor-pregna-1,3,5(10),6,8-pentaene-3,17α,21-triol-20-one 21-acetate;
5) 6-trifluoromethyl-19-nor-pregna-1,3,5(10),6,8-pentaene-3,17α,21-triol-20-one 21-acetate;
6) 19-nor-pregna-1,3,5(10),6,8-pentaene-3,16α,17α,21-tetrol-20-one 16,21-diacetate;
7) 6-fluoro-16α,17α-isopropylidenedioxy-19-nor-pregna-1,3,5(10),6,8-pentaene-3,21-diol-20-one 21-acetate;
8) 6-fluoro-16α-methyl-19-nor-pregna-1,3,5(10),6,8-pentaene-3,17α,21-triol-20-one 21-acetate;
9) 6-methyl-19-nor-pregna-1,3,5(10),6,8-pentaene-3,17α,21-triol-20-one 21-acetate;
10) 6,16β-dimethyl-19-nor-pregna-1,3,5(10), 6,8-pentaene-3,17α,21-triol-20-one 21-acetate.

B. In a manner similar to that described in Example 1B, treat each of the 19-nor-pregna-1,3,5(10),6,8-pentaenes obtainable from Example 8A with DDQ in dioxane and isolate and purify each of the resultant products, to obtain, respectively,

1) 6,16α-dimethyl-19-nor-pregna-1,3,5(10),6,8,14-hexaene-3,17α,21-triol-20-one 21-acetate;
2) 6-fluoro-19-nor-pregna-1,3,5(10),6,8,14-hexaene-3,17α,21-triol-20-one 21-acetate;
3) 6-fluoromethyl-19-nor-pregna-1,3,5(10),6,8,14-hexaene-3,17α,21-triol-20-one 21-acetate;
4) 6-difluoromethyl-19-nor-pregna-1,3,5(10),6,8,14-hexaene-3,17α,21-triol-20-one 21-acetate;
5) 6-trifluoromethyl-19-nor-pregna-1,3,5(10),6,8,14-hexaene-3,17α,21-triol-20-one 21-acetate;
6) 19-nor-pregna-1,3,5(10),6,8,14-hexaene-3,16α,17α,21-tetrol-20-one 16,21-diacetate;
7) 6-fluoro-16α, 17α-isopropylidenedioxy-19-nor-pregna-1,3,5(10),6,8,14-hexaene-3,21-diol-20-one 21-acetate;
8. 6-fluoro-16α-methyl-19-nor-pregna-1,3,5(10),6,8,14-hexaene-3,17α,21-triol-20-one 21-acetate;
9) 6-methyl-19-nor-pregna-1,3,5(10),6,8,14-hexaene-3,17α,21-triol-20-one 21-acetate;
10) 6,16β-dimethyl-19-nor-pregna-1,3,5(10),6,8,14-hexaene-3,17α,21-triol-20-one 21-acetate.

## EXAMPLE 18

### ALTERNATE PREPARATION OF 16α-METHYL-19-NOR-PREGNA-1,3,5(10),6,8,14-HEXAENE-3,17α,21-TRIOL-20-ONE 21-ACETATE

To a solution of 9α,11β-dichloro-16α-methyl-1,4-pregnadiene-17α,21-diol-3,20-dione 21-acetate (31 g) in dioxane (1.8 liters), add a solution of hydrogen chloride gas (66 g) in dioxane (420 ml) and DDQ (18.75 g). Stir the reaction mixture on a steam bath for 48 hours, then at room temperature for 40 hours. Concentrate the reaction mixture *in vacuo* to a low volume, then chromatograph the resultant residue over alumina (activity V), eluting with ether. Evaporate the combined eluates and recrystallize the resultant residue from acetone:hexane, to obtain 16α-methyl-19-nor-pregna-1,3,5(10),6,8,14-hexaene-3,17α,21-triol-20-one 21-acetate.

## EXAMPLE 19

### ALTERNATE PREPARATION OF 16α-METHYL-19-NOR-PREGNA-1,3,5(10),6,8,14-HEXAENE-3,17α,21-TRIOL-20-ONE 3,21-DIACETATE

To a solution of 16α-methyl-pregna-1,4,6,8,14-pentaene-17α,21-diol-3,20-dione 21-acetate

(0.075 g) and lithium chloride (0.075 g) in dimethylformamide (2 ml), add one drop of concentrated hydrochloric acid and reflux for half hour, then remove the solvent *in vacuo,* dissolve the residue in a small amount of acetone, and add a large quantity of water. Purify the resulting product by thin-layer chromatography, elution of the least polar band with acetone and removal of the solvent. Treat for 16 hours with pyridine (1 ml) and acetic anhydride (0.5 ml), add water, and recrystallize the resulting precipitate from acetone/hexane, to obtain 16α-methyl-19-nor-pregna-1,3,5(10),6,8,14-hexaene-3,17α,21-triol-20-one 3,21-diacetate.

The method-of-use aspect of this invention resides in eliciting a mitotic inhibitory response in a warm-blooded animal having a disease characterized by rapid cell proliferation, which comprises administering to said animal a non-toxic, mitotic-inhibitory effective amount of a 19-nor-pregnahexaene-20-one of formula (I) defined hereinabove, usually together with a non-toxic, pharmaceutically acceptable carrier, particularly in the treatment and control of proliferative skin diseases, primarily for the treatment of psoriasis via the topical route.

Psoriasis is characterized by increased epidermipoiesis associated with a high mitotic rate, rapid cell turnover and altered keratinization. The psoriatic epidermis can be normalized by slowing down cell growth through inhibiting mitosis. All drugs currently used in psoriasis therapy are known to directly or indirectly reduce epidermal mitotic activity. Although there is no animal model for psoriasis, many of these same drugs have been reported to have a similar effect in models of epidermal hyperplasia which simulate psoriasis in laboratory animals. Topically effective anti-psoriatic drugs, including corticosteroids, anthralin, coal tar and 5-fluoroacil, while relatively free of systemic side effects, cause local adverse reactions. Thus, corticosteroid therapy causes skin atrophy, telangiectasia and the formation of striae, while anthralin and 5-fluorouracil are skin irritants and require close clinical supervision for optimal therapeutic benefit. Anthralin can also cause staining of the skin.

It has now been discovered that compounds according to general formula (I) [particularly 16α-methyl-19-nor-pregna-1,3,5(10),6,8,14-hexaene-3,17α,21-triol-20-one 21-acetate] reduce epidermal mitotic activity without causing significant local or systemic hormonal or toxic effects when applied topically to the skin of mice in which epidermal mitosis has been stimulated.

Specifically, when treated by a procedure modified from S. Belman and W. Troll, Cancer Research *32*:450—454 (1972), the 19-nor-pregnahexaene-20-ones of this invention, particularly the 16α-methyl derivatives of formula (II), reduce croton-oil stimulated epidermal mitosis in mice when applied topically. Moreover, they are non-irritating without causing hormonal side effects, which is surprising in view of the 19-nor-pregnahexaene-20-one structure containing an aromatic A-ring such as in many estrogens, and a corticoid side chain such as in potent topical anti-inflammatory agents.

In the foregoing test, croton-oil is applied topically to shaved mice, thus accelerating mitosis. A 19-nor-pregna-1,3,5(10),6,8,14-hexaene-3,17α,21-triol-20-one of this invention is applied topically to the stimulated site, then 24 hours later portions of the treated skin are excised for histologic processing, mitotic figures per thousand basal interfollicular epidermal cells being counted in a light microscope. Epidermal mitotic counts from treated mice are compared to counts from lesion controls for statistically significant differences with an analysis of variance. The mitotic count for each compound tested is expressed as percent reduction of mitoses compared with the number of mitoses on the skin of mice treated with croton-oil alone. In general, it was discovered that the 19-nor-pregnahexaene-20-ones of this invention (formula I) significantly reduce croton-oil stimulated epidermal mitosis. For example, the 16α-methyl-19-nor-pregnahexaene-20-one of formula (II) usually exhibit over 60% inhibitions of mitoses at a 2 mg topical dose. 16α-Methyl-19-nor-pregna-1,3,5(10),6,8,14-hexaene-3,17α,21-triol-20-one 21-acetate (a preferred compound of the invention) exhibits about 80% reduction of mitosis (even at a topical dose as low as 0.2 mg) which is approximately 10 times greater than the mitotic reduction exhibited by an equal quantity (i.e. 0.2 mg) of betamethasone dipropionate (a known anti-psoriatic agent) in the same animal model.

The anti-mitotic activity is also demonstrated by similar tests in mice whereby increased epidermal mitosis is produced by ultraviolet irradiation according to procedures modified from A. DuVivier and R. B. Stoughton, J. Investigative Dermatology, *65*:233—237 (1975). In these tests, it was demonstrated that the 19-nor-pregnahexaene-20-ones of this invention, particularly 16α-methyl-19-nor-pregna-1,3,5(10),6,8,14-hexaene-3,17α,21-triol-20-one 21-acetate, significantly reduce epidermal mitotic rate following 1, 5 or 9 topical applications (each with 0.02 mg, 0.10 mg, and 0.5 mg doses) to ultraviolet stimulated hairless mouse epidermis, advantageously causing an epidermal thinning effect after multiple applications when the effect became equivalent to that demonstrated by steroidal anti-psoriatic agents such as betamethasone valerate. Since the compounds of this invention such as defined by formulae (I) and (II) do not cause estrogenic or other hormonal or toxic effects when applied topically as demonstrated by tests in mice, continued applications of a 19-nor-pregnahexaene-20-one of this invention will not cause irritation or staining of the skin or skin atrophy as caused by known anti-psoriatic agents. The foregoing mode of anti-psoriatic activity of the 19-nor-pregnahexaene-20-ones of this invention is different from that demonstrated by known steroidal anti-psoriatic agents such as betamethasone valerate which, when applied topically to ultraviolet stimulated hairless mouse epidermis at doses equal to those of our 19-nor-pregnahexaene-20-ones [e.g. 16α-methyl-19-nor-pregna-1,3,5(10),6,8,14-hexaene-3,17α,21-triol-20-one-21-acetate] first cause an

epidermal thinning without reduction of mitoses.

The 19-nor-pregnahexaene-20-ones of this invention, particularly 16α-methyl-19-nor-pregna-1,3,5(10),6,8,14-hexaene-3,17α,21-triol-20-one 21-acetate, have also been found to exhibit anti-mitotic activity when administered orally or parenterally to mice, without causing significant local or systemic hormonal or toxic effects.

In view of the anti-mitotic and anti-acanthotic (i.e., reduction of epidermal thickening) activity (as tested in mice), of the 19-nor-pregnahexaene-20-ones of this invention, particularly when applied topically, the invention includes the concept of treating and controlling psoriasis which comprises applying topically to the affected area, in a concentration effective for the treatment of psoriasis, a 19-nor-pregnahexaene-20-one of formula (I), usefully together with a non-toxic pharmaceutically acceptable carrier. Preferred anti-psoriatic agents of this invention are the 16-methyl-19-nor-pregnahexaene-20-ones, especially the 16α-methyl compounds of formula (II), particularly 16α-methyl-19-nor-pregna-1,3,5(10),6,8,14-hexaene-3,17α,21-triol-20-one 21-acetate and the 3-acetate, 3-benzoate and 15 chloro derivatives thereof.

Included within the term "topically applying" are applications onto the skin surface whereby the compounds of the invention are effective in the treatment and control of skin diseases characterized by rapid and/or abnormal cell proliferation, e.g. psoriasis; aerosol application; and subcutaneous injection application whereby they are effective in the treatment of local epidermal disorders.

Conveniently, a pharmaceutical formulation comprising a 19-nor-pregnahexaene-20-one of formula (I), preferably a 16α-methyl compound of formula (II), such as 16α-methyl-19-nor-pregna-1,3,5(10),6,8,14-hexaene-3,17α,21-triol-20-one 21-acetate, in a non-toxic pharmaceutically acceptable carrier, usually in concentrations from about 0.0001 percent to about 5 percent, preferably from about 0.1 percent to about one percent, is applied several times daily to skin affected by psoriasis until the psoriatic condition has improved. Topical applications may then be continued at less frequent intervals (e.g. once a day) to control mitoses in order to prevent return of severe psoriatic conditions. In general, application is in any topical form including creams, lotions, aerosols and ointments, prepared by combining the active ingredient with conventional pharmaceutical diluents and carriers as used in topical formulations comprising steroids; conveniently in a liquid solvent, preferably in a water-miscible liquid carrier made up of hydrophylic liquids having a high solvating action, e.g. a solution of 16α-methyl-19-nor-pregna-1,3,5(10),6,8,14-hexaene-3,17α,21-triol-20-one 21-acetate in polyethyleneglycol.

Thus, the pharmaceutical formulation aspect of this invention resides in the concept of a pharmaceutical composition, preferably for topical application, comprising an anti-psoriatically effective amount of a 19-nor-pregnahexaene-20-one of formula (I) together with a non-toxic pharmaceutically acceptable carrier. Preferred are topical formulations comprising 16α-methyl-19-nor-pregnahexaene-20-ones of formula (II), particularly 16α-methyl derivatives thereof having a free hydroxyl function at C—17, pharmaceutical formulations comprising 16α-methyl-19-nor-pregna-1,3,5(10),6,8,14-hexaene-3,17α-21,triol-20-one 21-acetate being particularly valuable.

The pharmaceutical formulations may be made according to known procedures, some of which are described in detail hereinbelow. Typical formulations include ointments, lotions, creams, sprays, powders, drops (e.g. ear drops), suppositories, and aerosols. Ointments and creams may, for example, be formulated with an aqueous or oily base with the addition of suitable thickening and/or gelling agents. Such bases may, thus, for example, include water and/or an oil (such as liquid paraffin) or a vegetable oil (such as peanut oil or castor oil). Thickening agents which may be used according to the nature of the base include soft paraffin, aluminium stearate, cetostearyl-alcohol, polyethyleneglycols, woolfat, hydrogenated lanolin, beeswax, etc. Lotions may be formulated with an aqueous or oily base and will, in general, also include one or more of the following, namely, stabilizing agents, emulsifying agents, dispersing agents, suspending agents, thickening agents, coloring agents, perfumes and the like. Powders may be formed with the aid of any suitable powder base, e.g. talc, lactose, starch, etc. Drops may be formulated with an aqueous base or non-aqueous base, also comprising one or more dispersing agents, suspending agents, solubilizing agents, etc.

The topical pharmaceutical compositions according to the invention may also include one or more preservatives or bacteriostatic agents, e.g. methyl hydroxybenzoate, propyl hydroxybenzoate, chlorocresol, benzalkonium chloride, etc. They may also contain other active ingredients such as antimicrobial agents, particularly antibiotics.

The inventive concept also includes pharmaceutical formulations for oral or parenteral administration, obtainable by standard procedures and comprising an anti-mitotic amount of a 19-nor-pregna-1,3,5(10),6,8,14-hexaene-20-one of formula (I) together with a non-toxic, pharmaceutically acceptable carrier.

The proportion of active steroid in the topical compositions according to the invention depends on the precise type of formulations to be prepared, but will generally be within the range of from 0.0001%d to 5% by weight. Generally, however, for most types of topical preparations the proportions of active steroid used will be within the range of from 0.1 to 3% and preferably 0.1 to 1%. Based upon studies in mice, when administered systemically, preferably parenterally, the dosage necessary to

produce an anti-mitotic response is in the range of from about 1 to about 100 mg per kilogram body weight.

The following Formulations exemplify some of the dosage forms in which the anti-mitotic agents of our invention may be employed. In each Formulation, the active ingredient is 16α-methyl-19-nor-pregna-1,3,5(10),6,8,14-hexaene-3,17α,21-triol-20-one 21-acetate. It will be appreciated, however, that this compound is but a representative example and may be replaced by equivalent quantities of other active compounds of this invention, e.g. by its 3-acetate, 3-benzoate or 15-chloro derivative.

FORMULATIONS

Formulation I: Ointment

| Formula | |
|---|---|
| 16α-Methyl-19-nor-pregna-1,3,5(10),6,8,14-hexaene-3,17α,21-triol-20-one 21-acetate, micronized | 1.0—20.0 mg |
| Benzyl alcohol, NF* | 10.0 mg |
| Mineral oil, USP | 50.0 mg |
| White petrolatum, USP, to make | 1.0 g |

Procedure

Mix and heat to 65°C a weighed quantity of white petrolatum, mineral oil, benzyl alcohol, and cool to 50—55°C with stirring. Disperse active ingredient in a portion of the mineral oil and then add to the above mixture with stirring. Cool to room temperature.

Formulation II: Cream

| Formula | |
|---|---|
| 16α-Methyl-19-nor-pregna-1,3,5(10),6,8,14-hexaene-3,17α,21-triol-20-one 21-acetate | 1.0—20.0 mg |
| Stearic acid, USP | 60.0 mg |
| Glyceryl monostearate, cosmetic grade | 100.0 mg |
| Propenyleneglycol, USP | 50.0 mg |
| Polyethylene-sorbitan monopalmitate | 50.0 mg |
| Sorbitol solution, USP | 30.0 mg |
| Benzyl alcohol, NF | 10.0 mg |
| Purified water, USP, to make | 1.0 g |

Procedure

Heat the stearic acid, glyceryl monostearate and polyethylene-sorbitan monopalmitate to 70°C. In a separate vessel, dissolve sorbitol solution, benzyl alcohol, water, and half quantity of propyleneglycol and heat to 70°C. Add the aqueous phase to the oily phase with high-speed stirring, allow resulting emulsion to gradually cool. Dissolve active ingredient in remaining quantity of propyleneglycol and add resulting solution to the above emulsion when the temperature of the latter is 37—40°C. Mix uniformly with stirring and cool to room temperature.

*) this refers to the requirements of the (U.S.) National Formulary

Formulation III: Gel

| Formula | |
|---|---|
| 16$\alpha$-Methyl-19-nor-pregna-1,3,5(10),6,8,14-hexaene-3,17$\alpha$,21-triol-20-one 21-acetate | 1.0—20.0 mg |
| Propyleneglycol, USP | 300.0 mg |
| 3,5-di-(tert.-butyl)-4-hydroxy-toluene ("butylated hydroxytoluene") | 5.0 mg |
| Carbomer 940* | 5.0 mg |
| Sodium hydroxide (added as a 1% w/w solution in propyleneglycol | 0.7 mg |
| Polyethyleneglycol 400 (PEG—8*), USP | 669.3—668.3 mg |

*) see CTFA Dictionary

Procedure

Prepare a 1% solution of the sodium hydroxide in propyleneglycol and set aside. Separately, mix approximately one-half the remaining propyleneglycol and the polyethyleneglycol 400, then dissolve the butylated hydroxytoluene in this mixture. Disperse the Carbomer 940 in the foregoing mixture with vigorous agitation, then add the sodium hydroxide solution with high-speed agitation to bring the pH of the solution up to 7. Continue stirring until a thick gel forms. Dissolve the active ingredient in the remaining propyleneglycol and add the resulting solution to the gel slowly, with continuous stirring.

Formulation IV: Lotion

| Formula | |
|---|---|
| 16$\alpha$-Methyl-19-nor-pregna-1,3,5(10),6,8,14-hexaene-3,17$\alpha$,21-triol-20-one 21-acetate | 1.0—20.0 mg |
| Carbomer 940 (G. W. Goodrich) | 3.0 mg |
| Sodium hydroxide (charged as 4% w/w aqueous solution) | 0.05 mg |
| Isopropanol | 40.00 mg |
| Purified water, USP, to make | 1.0 g |

Procedure

Prepare 4% aqueous sodium hydroxide solution and hold. Heat the purified water to 60°C, add Carbomer 940, and mix at high speed until dispersed. Cool dispersion to room temperature and slowly charge sodium hydroxide solution until uniform. Add 80% of the isopropanol to the above with mixing. Dissolve active ingredient in remaining isopropanol and add to mixture with stirring. Adjust pH to 5.0 to 5.5 with further sodium hydroxide, if necessary.

Formulation V: Tablets

| Formula | 10 mg Tab. | 25 mg Tab. | 100 mg Tab. |
|---|---|---|---|
| 16$\alpha$-Methyl-19-nor-pregna-1,3,5(10),6,8,14-hexaene-3,17$\alpha$,21-triol-20-one 21-acetate | 10.5* mg | 26.5* mg | 105.0* mg |
| Lactose, impalpable powder | 197.50 mg | 171.25 mg | 126.00 mg |
| Corn starch | 25.00 mg | 25.00 mg | 35.00 mg |
| Polyvinylpyrrolidone | 7.50 mg | 7.50 mg | 7.50 mg |
| Magnesium stearate | 2.50 mg | 2.50 mg | 3.50 mg |

*5% excess

Procedure

Prepare a slurry consisting of active ingredient, lactose and polyvinylpyrrolidone. Spray dry the slurry. Add the corn starch and magnesium stearate. Mix and compress into tablets.

Formulation VI: Parenteral Compositions

A. Intramuscular or Subcutaneous Oil Injection

| Formula | |
|---|---|
| 16$\alpha$-Methyl-19-nor-pregna-1,3,5(10),6,8,14-hexaene-3,17$\alpha$,21-triol-20-one 21-acetate | 1—20 mg |
| Aluminium monostearate, USP | 20.0 mg |
| Propylbaren, USP | 1.0 mg |
| Sesame oil, USP (heat treated), to make | 1.0 ml |

Procedure

The other ingredients are dissolved in sesame oil and brought to a total volume of 1 ml.

B. Intramuscular or Subcutaneous Aqueous Suspension

| Formula | |
|---|---|
| 16$\alpha$-Methyl-19-nor-pregna-1,3,5(10),6,8,14-hexaene-3,17$\alpha$,21-triol-20-one 21-acetate | 1—20 mg |
| Monobasic sodium phosphate | 6.0 mg |
| Dibasic sodium phosphate, anhydrous | 0.5 mg |
| Polysorbate 80*, USP | 0.05 mg |
| Benzyl alcohol, reagent grade | 9.0 mg |
| Methylparaben, USP | 1.3 mg |
| Propylparaben, USP | 0.2 mg |
| Sodium chloride, USP | 2.5 mg |
| Sodium carboxymethylcellulose, USP | 3.0 mg |
| Ethylenediamine-tetracetate, disodiuim salt, USP | 0.1 mg |
| Water for injection, USP, to make | 1.0 ml |

*) see CTFA Dictionary

Procedure

The other ingredients are dissolved in water and brought to a total volume of 1 ml.

**Claims**

1. 19-Nor-pregnahexaene derivatives of the general formula

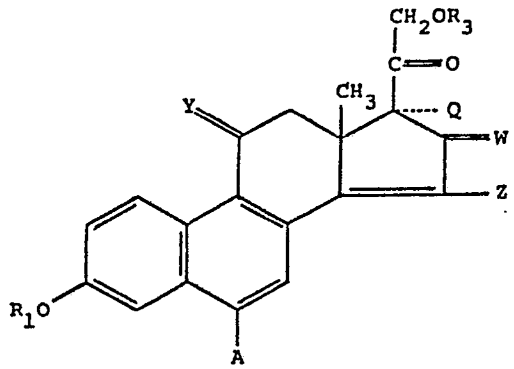

(I)

wherein

A is hydrogen, lower alkyl, fluoro or fluoro-substituted methyl;

$R_1$ is hydrogen, lower alkyl, or an acyl radical of a carboxylic acid having up to 12, preferably up to 8, carbon atoms;

W is (H,H); (H, lower alkyl); (H, $\alpha$-$OR_2$), with $R_2$ being hydrogen or an acyl radical of a carboxylic acid having up to 12, preferably up to 8, carbon atoms; or =CHT, with T being hydrogen, lower alkyl, fluorine, or chlorine;

Q is $OR_4$, with $R_4$ being hydrogen or an acyl radical of a carboxylic acid having up to 12, preferably up to 8, carbon atoms; hydrogen, provided W is (H,H) or (H, lower alkyl); or together with W represents a 16$\alpha$,17$\alpha$-lower alkylidenedioxy grouping;

Y is (H,H), (H, OH), or oxygen;

Z is hydrogen, chlorine or bromine;

$R_3$ is hydrogen or an acyl radical of a carboxylic acid having up to 12, preferably up to 8, carbon atoms; or $OR_3$ together with Q represents an alkylidenedioxy or alkylorthoalkanoate grouping; and when Q is hydroxy and $R_3$ is hydrogen, the 17$\alpha$,20; 20,21-bismethylenedioxy derivatives thereof; with the exception of the 21-acetate and the 3,21-diacetate of 19-nor-pregna-1,3,5(10),6,8,14-hexaene-3,17$\alpha$,21-triol-20-one.

2. Pharmaceutical compositions containing, as an active ingredient, a compound of the general formula (I), as defined in claim 1, inclusive those compounds referred to in said claim as being excepted.

3. The compounds and compositions of claims 1 and 2, respectively, wherein W in formula (I) represents (H, $\alpha$-methyl).

4. The compounds and compositions of claim 3, wherein Q is $OR_4$ ($R_4$ being as defined in claim 1); each of A and Z is hydrogen;

Y represents (H,H);

$R_1$ is as defined in claim 1; and

$R_3$ is hydrogen or an acyl radical of a carboxylic acid having up to 12, preferably up to 8, carbon atoms.

5. 16$\alpha$-Methyl-19-nor-pregna-1,3,5(10),6,8,14-hexaene-3,17$\alpha$,21-triol-20-one 21-acetate, and pharmaceutical compositions containing the same.

6. The pharmaceutical compositions of any of claims 2 to 5, also containing a suitable pharmaceutical carrier, for the use of eliciting a mitotic-inhibitory response in a warm-blooded animal having a disease characterized by rapid cell proliferation, especially for the treatment of psoriasis.

7. Process for the preparation of the 19-nor-pregnahexaene derivatives of claim 1, characterized in that

(A) an appropriate 19-nor-pregna-1,3,5(10),6,8-pentaene derivative is subjected to dehydrogenation in position 14(15); or

(B) an appropriate 11-unsubstituted pregna-1,4,6,8,14-pentaene-3-one is subjected to aromatization; or

(C) an appropriate 9$\alpha$,11$\beta$-dihalogeno-pregna-1,4,6-triene-3-one is subjected to concomitant didehydrohalogenation and aromatization; it being understood that aromatization O as per (B) and (C) inherently involves removal of the angular methyl at C—10; and the resulting 19-nor-pregna-13,5(10),6,8,14-hexaene derivative, if desired, is subjected to one or more of the following facultative finishing steps:

(i) de-esterification and/or esterification at hydroxyl functions in one or more of positions 3, 11$\beta$, 16$\alpha$, 17$\alpha$ and 21;

(ii) alkylation and/or dealkylation at the hydroxyl function in position 3;

(iii) introduction or removal of a 16$\alpha$,17$\alpha$- or 17$\alpha$,21-alkylidenedioxy or of a 17$\alpha$,20; 20,21-bismethylenedioxy function;

(iv) reduction or oxidation of an 11-oxygen function;

(v) chlorination or bromination in position 15.

8. Process according to claim 7A, characterized in that the dehydrogenation is performed utilizing at least one molar equivalent of 2,3-dichloro-5,6-dicyanobenzoquinone as dehydrogenating agent, in an aprotic solvent.

9. Process according to claim 8, for the preparation of such compounds of formula (I) wherein Z is a chlorine or bromine atom, characterized in that the dehydrogenation of the 19-nor-pregna-1,3,5(10),6,8-pentaene starting compound unsubstituted in position 14 is performed, utilizing at least two molar equivalents of 2,3-dichloro-5,6-dicyanobenzoquinone, in the presence of at least one molar equivalent of hydrogen chloride or hydrogen bromide, so as to achieve concomitant 15-halogenation.

10. Process according to claim 7B, characterized in that aromatization is performed by means of a weak base, desirably in the presence of a soluble halide salt such as lithium chloride, preferably by means of lithium chloride/dimethylformamide.

11. Process according to claim 7C, characterized in that an appropriate 9$\alpha$,11$\beta$-dichloro-pregna-1,4-diene-3-one is subjected to concomitant 6-dehydrogenation, didehydrochlorination and aromatization.

12. Process according to claim 11, characterized in that the reactions are performed in situ at elevated temperatures, by means of 2,3-dichloro-5,6-dicyanobernzoquinone as dehydrogenating agent and in the presence of an acid in an aprotic solvent.

**Patentansprüche**

1. 19-Nor-pregnahexaenderivate der allgemeinen Formel

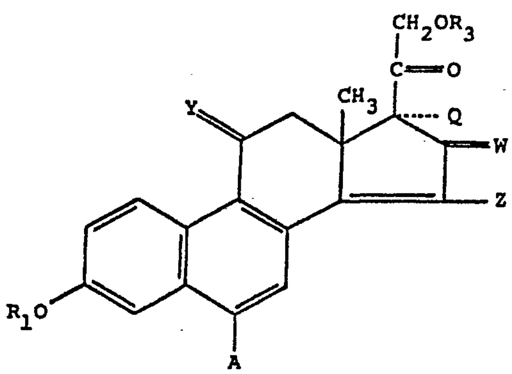

(I),

worin

A für Wasserstoff, einen niederen Alkylrest, Fluor oder einen fluorsubstituierten Methylrest,

$R_1$ für Wasserstoff, einen niederen Alkylrest oder einen Acylrest einer Carbonsäure mit bis zu 12 C-Atomen, vorzugsweise mit bis zu 8 C-Atomen,

W für (H,H), (H, neideres Alkyl), (H, $\alpha$-$OR_2$), worin $R_2$ Wasserstoff oder ein Acylrest einer Carbonsäure mit bis zu 12 C-Atomen, vorzugsweise mit bis zu 8 C-Atomen ist, oder =CHT steht, worin T Wasserstoff, niederes Alkyl, Fluor oder Chlor ist,

Q für $OR_4$, worin $R_4$ Wasserstoff oder ein Acylrest einer Carbonsäure mit bis zu 12 C-Atomen, vorzugsweise mit bis zu 8 C-Atomen ist, oder vorausgesetzt, dass W für (H,H) oder (H, niederes Alkyl) steht, für Wasserstoff steht oder zusammen mit W eine 16$\alpha$,17$\alpha$-Niederalkylidendioxygruppe darstellt,

Y für (H,H), (H, OH) oder Sauerstoff,

Z für Wasserstoff, Chlor oder Brom,

$R_3$ für Wasserstoff oder einen Acylrest einer Carbonsäure mit bis zu 12 C-Atomen, vorzugsweise mit bis zu 8 C-Atomen steht oder $OR_3$ zusammen mit Q eine Alkylidendioxy- oder Alkylorthoalkanoatgruppe darstellt, und, wenn Q eine Hydroxylgruppe und $R_3$ Wasserstoff sind, ihre 17$\alpha$,20; 20,21-Bismethylendioxyderivate; mit Ausnahme des 21-Acetats und des 3,21-Diacetats von 19-Nor-pregna-1,3,5(10),6,8,14-hexaen-3,17$\alpha$,21-triol-20-on.

2. Arznneimittelzubereitungen, enthaltend als aktives Ingrediens eine Verbindung der allgemeinen Formel (I) gemäss Anspruch 1, einschliesslich der in Anspruch 1 als ausgenommen bezeichneten Verbindungen.

3. Verbindungen und Arzneimittelzubereitungen nach Anspruch 1 bzw. 2, dadurch gekennzeichnet, daß W in der Formel (I) für (H, $\alpha$-Methyl) steht.

4. Verbindungen und Zubereitungen nach Anspruch 3, dadurch gekennzeichnet, daß Q für $OR_4$ (worin $R_4$ die in Anspruch 1 genannte Bedeutung hat) steht, A und Z jeweils Wasserstoff sind, Y für (H,H) steht, $R_1$ die in Anspruch 1 genannte Bedeutung hat und $R_3$ Wasserstoff oder ein Acylrest einer Carbonsäure mit bis zu 12 C-Atomen, vorzugsweise mit bis zu 8 C-Atomen ist.

5. 16$\alpha$-Methyl-19-nor-pregna-1,3,5(10),6,8,14-hexaen-3,17$\alpha$,21-triol-20-on-21-acetat und es enthaltende Arzneimittelzubereitungen.

6. Arzneimittelzubereitungen nach einem der Ansprüche 2 bis 5, die ausserdem einen geeigneten pharmazeutischen Träger enthalten, für die Verwendung zum Hervorrufen einer Hemmung der Mitose bei Warmblütern, die von einer Krankheit befallen sind, die durch schnelle Proliferation der Zellen gekennzeichnet ist, insbesondere für die Behandlung der Psoriasis.

7. Verfahren zur Herstellung von 19-Nor-pregnahexaenderivaten nach Anspruch 1, dadurch gekennzeichnet, daß man

(A) ein geeignetes 19-Nor-pregna-1,3,5(10),6,8-pentaenderivat der Dehydrierung in der 14(15)-Stellung oder

(B) ein geeignetes 11-unsubstituiertes Pregna-1,4,6,8,14-pentaen-3-on der Aromatisierung oder

(C) ein geeignetes 9$\alpha$,11$\beta$-Dihalogen-pregna-1,4,6-trien-3-on der gleichzeitigen Didehydrohalogenierung und Aromatisierung unterwirft,

wobei durch die Aromatisierung in der Stufe (B) und (C) zwangsläufig der angulare Methylrest bei C—10 entfernt wird, und gegebenenfalls das erhaltene 19-Nor-pregna-1,3,5(10), 6,8,14-hexaenderivat einer oder mehreren der folgenden fakultativen Nachbehandlungen unterwirft:

(I) Entesterung und/oder Veresterung an Hydroxylfunktionen in einer oder mehreren der Stellungen 3, 11$\beta$, 16$\alpha$, 17$\alpha$ und 21,

(II) Alkylierung und/oder Dealkylierung an der Hydroxylfunktion in 3-Stellung,

(III) Einführung oder Entfernung einer 16$\alpha$, 17$\alpha$- oder 17$\alpha$, 21-Alkylidendioxyfunktion oder einer 17$\alpha$, 20; 20, 21-Bis-methylendioxyfunktion,

(IV) Reduktion oder Oxydation einer 11-Sauerstoffunktion und

(V) Chlorierung oder Bromierung in 15-Stellung.

8. Verfahren nach Anspruch 7A, dadurch gekennzeichnet, daß man die Dehydrierung unter Verwendung von wenigstens einem molaren Äquivalent von 2,3-Dichlor-5,6-dicyanbenzochinon als Dehydrierungsmittel in einem aprotischen Lösungsmittel durchführt.

9. Verfahren nach Anspruch 8 zur Herstellung von Verbindungen der Formel (I), worin Z ein Chloratom oder Bromatom ist, dadurch gekennzeichnet, daß man die Dehydrierung des als Ausgangsmaterial verwendeten in 14-Stellung unsubstituierten 19-Nor-pregna-1,3,5(10),6,8,-pentaens unter Verwendung von wenigstens zwei molaren Äquivalenten von 2,3-Dichlor-5,6-dicyanbenzochinon in Gegenwart von wenigstens einem molaren Äquivalent von Chlorwasserstoff oder Bromwasserstoff durchführt, wobei gleichzeitige 15-Halogenierung erreicht wird.

10. Verfahren nach Anspruch 7B, dadurch gekennzeichnet, daß man die Aromatisierung mit Hilfe einer schwachen Base, zweckmäßig in Gegenwart eines löslichen Halogenidsalzes, z.B. Lithiumchlorid, vorzugsweise mit Hilfe von Lithiumchlorid/Dimethylformamid, durchführt.

11. Verfahren nach Anspruch 7C, dadurch gekennzeichnet, daß man ein geeignetes 9$\alpha$,11$\beta$-Dichlor-pregna-1,4-dien-3-on der gleichzeitigen 6-Dehydrierung, Didehydrochlorierung und Aromatisierung unterwirft.

12. Verfahren nach Anspruch 11, dadurch gekennzeichnet, daß man die Reaktionen in situ bei erhöhten Temperaturen mit Hilfe von 2,3-Dichlor-5,6-dicyanbenzochinon als Dehydrierungsmittel in Gegenwart einer Säure in einem aprotischen Lösungsmittel durchführt.

**Revendications**

1. Dérivés de 19-Nor-pregnahexaène de la formule générale:

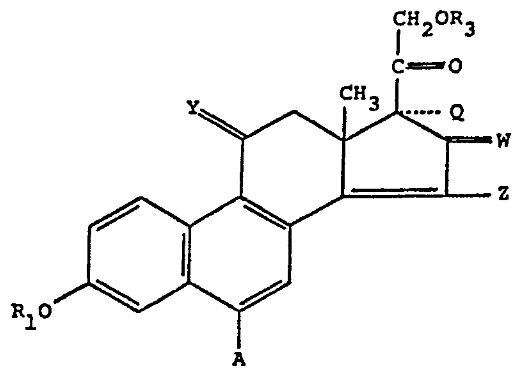

(I)

dans laquelle A représente l'hydrogène, un alcoyle inférieur, un fluoro ou un méthyl fluoro-substitué;

$R_1$ représente l'hydrogène, un alcoyle inférieur, ou un radical acyle d'un acide carboxylique ayant jusqu'à 12, de préférence jusqu'à 8, atomes de carbone;

W représente (H,H); (H, alcoyle inférieur); (H, $\alpha$-$OR_2$), avec $R_2$ étant l'hydrogène ou un radical acyle d'un acide carboxylique ayant jusqu'à 12, de préférence jusqu'à 8, atomes de carbone; ou = CHT, avec T représentant l'hydrogène, un alcoyle inférieur, du fluor, ou du chlore;

Q représente $OR_4$, avec $R_4$ étant l'hydrogène ou un radical acyle d'un acide carboxylique ayant jusqu'à 12, de préférence jusqu'à 8, atomes de carbone; l'hydrogène, pourvu que W représente (H,H) ou (H, alcoyle inférieur); ou avec W représente un groupement 16$\alpha$,17$\alpha$-alcoylidène inférieur dioxy;

Y représente (H,H), (H, OH) ou l'oxygène;

Z représente l'hydrogène, du chlore ou du brome;

$R_3$ représente l'hydrogène ou un radical acyle d'un acide carboxylique ayant jusqu'à 12, de préférence jusqu'à 8, atomes de carbone; ou $OR_3$ avec Q représente un groupement alcoylidènedioxy ou alcoylorthoalcanoate;

et lorsque Q représente un hydroxy et $R_3$ représente l'hydrogène, les dérivés 17$\alpha$,20; 20,21-bisméthylènedioxy de ceux-ci;

à l'exception du 21-acétate et du 3,21-diacétate de la 19-Nor-pregna-1,3,5(10),6,8,14-hexaène-3,17$\alpha$,21-triol-20-one.

2. Compositions pharmaceutiques contenant, comme ingrédient actif, un composé de la formule générale (I), tel que défini à la revendication 1, y compris les composés rapportés dans cette revendication comme étant exceptés.

3. Les composés et compositions des revendications 1 et 2, respectivement, dans lesquelles W dans la formule (I) représente (H, $\alpha$-méthyle).

4. Les composés et compositions de la revendication 3, dans lesquels

Q représente $OR_4$ ($R_4$ étant tel que défini à la revendication 1);

chacun de A et Z représente l'hydrogène;

Y représente (H,H);

$R_1$ est tel que défini à la revendication 1; et

$R_3$ représente l'hydrogène ou un radical acyle d'un acide carboxylique ayant jusqu'à 12, de préférence jusqu'à 8, atomes de carbone.

5. Le 16$\alpha$-méthyl-19-nor-pregna-1,3,5(10),6,8,14-hexaène-3,17$\alpha$,21-triol-20-one 21-acétate, et les compositions pharmaceutiques le contenant.

6. Composition pharmaceutique selon l'une quelconque des revendications 2 à 5, contenant également un support pharmaceutique approprié, pour l'emploi de l'obtention d'une réponse inhibitoire mitotique dans un animal à sang chaud ayant une maladie caractérisée par une prolifération cellulaire rapide, spécialement pour le traitement de psoriasis.

7. Procédé de préparation des dérivés 19-nor-pregnahexaène de la revendication 1, caractérisé en ce que:

(A) un dérivé de 19-nor-pregna-1,3,5(10),6,8-pentaène approprié est soumis à une déshydrogénation en position 14(15); ou

(B) une 11-non-substituée pregna-1,4,6,8,14-pentaène-3-one appropriée est soumise à une aromatisation; ou

(C) une 9$\alpha$,11$\beta$-dihalogéno-pregna-1,4,6-triène-3-one appropriée est soumise à une didéshydrohalogénation et aromatisation concomitante;

étant entendu que l'aromatisation selon (B) et (C) inclut de manière inhérente l'enlèvement du méthyle angulaire en C—10; et le dérivé 19-nor-pregna-1,3,5(10),6,8,14-hexaène résultant, si désiré, est soumis à une ou plusieurs des étapes finales facultatives suivantes:

(i) une dé-estérification et/ou estérification aux fonctions hydroxyle dans une ou plusieurs des positions 3, 11$\beta$, 16$\alpha$, 17$\alpha$ et 21;

(ii) une alcoylation et/ou une déalcoylation à la fonction hydroxyle en position 3;

(iii) l'introduction ou l'enlèvement d'un 16$\alpha$,17$\alpha$- ou 17$\alpha$,21-alcoylidènedioxy ou d'une fonction 17$\alpha$,20; 20—21-bisméthylènedioxy;

(iiii) la réduction ou l'oxydation d'une fonction 11-oxygène

(iiiii) la chloration ou la bromation en position 15.

8. Procédé selon la revendication 7A, caractérisé en ce que la déshydrogénation est réalisée en utilisant au moins un équivalent molaire de 2,3-dichloro-5,6-dicyanobenzoquinone comme agent de déshydrogénation, dans un solvant aprotique.

9. Procédé selon la revendication 8, pour la préparation des composés de la formule (I) dans laquelle Z est un atome de chlore ou de brome, caractérisé en ce que la déshydrogénation du composé de départ 19-nor-pregna-1,3,5(10),6,8-pentaène non substitué en position 14 est réalisée, en utilisant au moins deux équivalents molaires de 2,3-dichloro-5,6-dicyanobenzoquinone, en présence d'au moins un équivalent molaire de chlorure d'hydrogène ou de bromure d'hydrogène, de façon à réaliser une 15-halogénation concomitante.

10. Procédé selon la revendication 7B, caractérisé en ce que l'aromatisation est réalisée au moyen d'une base faible, de manière désirable en présence d'un sel d'halogénure soluble tel que du chlorure de lithium, de préférence au moyen de chlorure de lithium/diméthylformamide.

11. Procédé selon la revendication 7C, caractérisé en ce qu'une 9$\alpha$,11$\beta$-dichloro-pregna-1,4-diène-3-one appropriée est soumise à une 6-déshydrogénation, didéshydrochloration et aromatisation concomitante.

12. Procédé selon la revendication 11, caractérisé en ce que les reactions sont réalisées in situ à des températures élevées, au moyen de 2,3-dichloro-5,6-dicyanobenzoquinone comme agent de déshydrogénation et en présence d'un acide dans un solvant aprotique.